(19) **Europäisches Patentamt / European Patent Office / Office européen des brevets**

(11) **EP 3 599 250 A1**

(12) **EUROPEAN PATENT APPLICATION**
published in accordance with Art. 153(4) EPC

(43) Date of publication:
**29.01.2020 Bulletin 2020/05**

(21) Application number: **18744269.4**

(22) Date of filing: **23.01.2018**

(51) Int Cl.:
*C07K 17/08* (2006.01)　　*C07K 16/46* (2006.01)
*A61K 39/44* (2006.01)　　*A61K 47/59* (2017.01)
*A61K 47/60* (2017.01)　　*A61P 35/00* (2006.01)
*A61P 37/02* (2006.01)　　*A61P 29/00* (2006.01)

(86) International application number:
**PCT/CN2018/073785**

(87) International publication number:
**WO 2018/137609 (02.08.2018 Gazette 2018/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **24.01.2017  CN 201710052692**
　　　　　　**21.06.2017  CN 201710480066**

(71) Applicant: **Benhealth Biopharmaceutic (Shenzhen) Co., Ltd.**
**Shenzhen 518055 (CN)**

(72) Inventors:
• **YU, Haoyang**
　**Shenzhen**
　**Guangdong 518055 (CN)**
• **WANG, Zhong**
　**Shenzhen**
　**Guangdong 518055 (CN)**
• **LI, Zhengcheng**
　**Shenzhen**
　**Guangdong 518055 (CN)**

(74) Representative: **Grünecker Patent- und Rechtsanwälte**
**PartG mbB**
**Leopoldstraße 4**
**80802 München (DE)**

(54) **ANTIBODY CONJUGATE, AND RELATED PHARMACEUTICAL COMPOSITION AND APPLICATION**

(57)　The present invention relates to a multi-specific antibody conjugate (such as, a bispecific antibody conjugate), and a related composition, therapeutic method and use thereof. The antibody conjugate comprises a multispecific antibody, such as a bispecific antibody, conjugated to a nanomaterial. The antibody conjugate can be used to modulate an immune response, treat or prevent a disease or condition (e.g., a cancer, autoimmune disease, pathogen infection or inflammatory disease), etc..

EP 3 599 250 A1

SEQUENCE LISTING

<110> BENHEALTH BIOPHARMACEUTIC (SHENZHEN) CO., LTD.

<120> ANTIBODY CONJUGATE, RELATED PHARMACEUTICAL COMPOSITION AND USE

<130> EP124561-SZ

<140> EP18744269.4
<141> 2018-01-23

<150> CN201710480066
<151> 2017-06-21

<150> CN201710052692
<151> 2017-01-24

<160> 5

<170> BiSSAP 1.3.6

<210> 1
<211> 119
<212> PRT
<213> Artificial Sequence

<220>
<223> Artificial Sequence

<400> 1
Glu Val Gln Leu Gln Glu Ser Gly Gly Gly Ser Val Gln Thr Gly Gly
1               5                   10                  15
Ser Leu Arg Leu Ser Cys Val Val Ser Gly Tyr Thr Tyr Ser Ser Ala
            20                  25                  30
Cys Met Gly Trp Phe Arg Gln Gly Pro Gly Lys Gly Arg Glu Ala Val
            35                  40                  45
Ala Asp Val Asn Thr Gly Gly Arg Arg Thr Tyr Tyr Ala Asp Ser Val
        50                  55                  60
Lys Gly Arg Phe Thr Ile Ser Gln Asp Glu Asn Thr Met Tyr Leu Gln
65                  70                  75                  80
Met Asn Asn Leu Lys Pro Glu Asp Thr Ala Thr Tyr Tyr Cys Ala Lys
                85                  90                  95
Gly Arg Cys Ile Ala Val Ala Gly Gly Pro Asp Tyr Trp Gly Gln Gly
            100                 105                 110
Thr Leu Val Thr Val Ser Ser
        115

<210> 2
<211> 122
<212> PRT
<213> Artificial Sequence

<220>
<223> Artificial Sequence

<400> 2
Glu Val Gln Leu Leu Glu Ser Gly Gly Gly Leu Val Gln Pro Gly Gly
1               5                   10                  15
Ser Leu Arg Leu Ser Cys Ala Ala Ser Gly Leu Thr Phe Ser Ser Tyr
            20                  25                  30
Asn Met Gly Trp Val Arg Gln Ala Pro Gly Lys Gly Leu Glu Trp Val

```
              35                        40                        45
      Ala Ser Ile Thr Trp Ser Gly Arg Asp Thr Phe Tyr Ala Asp Ser Val
              50                        55                        60
      Lys Gly Arg Phe Thr Ile Ser Arg Asp Asn Ser Lys Asn Thr Leu Tyr
      65                        70                        75              80
      Leu Gln Met Asn Ser Leu Arg Ala Glu Asp Thr Ala Val Tyr Tyr Cys
                      85                        90                        95
      Ala Ala Asn Pro Trp Pro Val Ala Ala Pro Arg Ser Gly Thr Tyr Trp
                      100                       105                       110
      Gly Gln Gly Thr Leu Val Thr Val Ser Ser
                      115                       120
```

```
<210> 3
<211> 20
<212> PRT
<213> Artificial Sequence


<220>
<223> Artificial Sequence


<400> 3
Met Lys Lys Ile Trp Leu Ala Leu Ala Gly Leu Val Leu Ala Phe Ser
1               5                   10                  15
Ala Ser Ala Ser
            20


<210> 4
<211> 121
<212> PRT
<213> Artificial Sequence


<220>
<223> Artificial Sequence


<400> 4
Leu Glu Glu Val Gln Leu Val Glu Ser Gly Gly Gly Leu Val Gln Pro
1               5                   10                  15
Gly Glu Ser Leu Thr Leu Ser Cys Val Val Ala Gly Ser Ile Phe Ser
                20                  25                  30
Phe Ala Met Ser Trp Tyr Arg Gln Ala Pro Gly Lys Glu Arg Glu Leu
                35                  40                  45
Val Ala Arg Ile Gly Ser Asp Asp Arg Val Thr Tyr Ala Asp Ser Val
            50                  55                  60
Lys Gly Arg Phe Thr Ile Ser Arg Asp Asn Ile Lys Arg Thr Ala Gly
65                  70                  75                  80
Leu Gln Met Asn Ser Leu Lys Pro Glu Asp Thr Ala Val Tyr Tyr Cys
                85                  90                  95
Asn Ala Gln Thr Asp Leu Arg Asp Trp Thr Val Arg Glu Tyr Trp Gly
                100                 105                 110
Gln Gly Thr Gln Val Thr Val Ser Ser
            115                 120


<210> 5
<211> 120
<212> PRT
<213> Artificial Sequence


<220>
<223> Artificial Sequence
```

```
<400> 5
Glu Val Gln Leu Val Glu Ser Gly Gly Gly Phe Val Gln Ala Gly Glu
1               5                   10              15
Ser Leu Thr Leu Ser Cys Thr Ser Ser Thr Leu Thr Phe Thr Pro Tyr
            20                  25                  30
Arg Met Ala Trp Tyr Arg Gln Ala Pro Gly Lys Gln Arg Asp Leu Val
        35                  40                  45
Ala Asp Ile Ser Ser Gly Asp Gly Arg Thr Thr Asn Tyr Ala Asp Phe
        50                  55                  60
Ala Lys Gly Arg Phe Thr Ile Ser Arg Asp Asn Ile Lys Asn Thr Val
65                  70                  75                  80
Phe Leu Arg Met Thr Asn Leu Lys Pro Glu Asp Thr Ala Val Tyr Tyr
                85                  90                  95
Cys Asn Thr Phe Val Ser Phe Val Gly Ile Ala Arg Ser Trp Gly Gln
            100                 105                 110
Gly Thr Gln Val Thr Val Ser Ser
            115                 120
```

**Description**

**Technical Field**

The present invention directs to the field of medical immunology, and more particularly to a multi-specific antibody conjugate, particularly a multispecific antibody conjugate for inducing an immune response to a disease such as a cancer or infectious disease. The invention also directs to a composition, application method and use related to the antibody conjugate.

**Background**

Some antibodies, such as multi-specific antibodies (such as bispecific antibodies), can bind to two or more different antigens. A number of recombinant strategies have been developed to synthesize a bispecific antibody, including a single-chain variable fragment (scFv)-derived form such as a diabody, a tandem diabody, BiTe (a bispecific T cell adaptor) and DART (double affinity re-targeting) as well as a form based on immunoglobulin G (IgG), such as Triomab, DVD-Ig (double variable domain antibody) and a two-in-one antibody. However, such bispecific antibody may have poor pharmacokinetic and physical properties (such as immunogenicity). Therefore, there is a need for an alternative technique that is able to improve the bispecific antibodies of the prior art.

**Summary of Invention**

A multispecific antibody conjugate is disclosed herein. The multispecific antibody conjugate described herein comprises a multispecific antibody conjugated to a nanomaterial, such as a nanoparticle.
The multispecific antibody preferably comprises at least one first binding moiety and at least one second binding moiety, wherein the first binding moiety binds to a cytotoxic effector cell, preferably an antigen on the cytotoxic effector cell, and the second binding moiety binds to a target cell, preferably an antigen on the target cell.
The cytotoxic effector cell can be a cytotoxic cell. The cytotoxic cell can be a leukocyte. The leukocyte may be selected from the group consisting of a macrophage, neutrophil, eosinophil, NK cell, B cell, and T cell. The leukocyte may be a T cell. The leukocyte can be a NK cell. The leukocyte may be a NK-like T cell, i.e. NKT cell. The leukocyte may be a NK cell and T cell. The cytotoxic effector cell can comprise a cell selected from the group consisting of a T cell, NK cell, and NKT cell. The cytotoxic effector cell can be selected from the group consisting of a T cell, NK cell, NKT cell, and CIK cell.
The target cell may be a cell to be cleared, and may be a diseased cell or disease-associated cell. For example, the target cell can be a cancer cell or a tumor cell. The target cell can be a leukemia cell. The target cell can be a lymphocyte. The target cell can be a metastatic cell. The target cell can be genetically modified. The target cell can contain gene mutation(s). The gene mutation(s) can include oncogene mutation(s). The gene mutation(s) can be mutation(s) of a tumor-suppressing gene. The gene mutation can be mutation(s) of the proto-oncogene. The target cell can be a cell associated with an inflammatory disease and/or an autoimmune disease. The target cell can be an infected cell. The target cell can be a pathogenic cell.
The multispecific antibody is preferably bispecific. Preferably, one binding site (the first binding site) of the multispecific antibody is directed to an antigen on a leukocyte, and the second binding site binds to an antigen on a target cell, which may be a diseased cell or disease-associated cell or pathogen (i.e., microorganism). The multispecific antibody may also be trispecific; preferably, wherein at least one binding site (the first binding site) is directed to an antigen on a leukocyte, and at least one second binding site binds to an antigen on a target cell, which may be a diseased cell or disease-associated cell or pathogen (i.e., microorganism).
An exemplary antigen on a T cell is selected from the group consisting of CD2, CD3, CD4, CD5, CD6, CD8, CD25, CD28, CD30, CD40, CD40L, CD44, CD45, CD69 and CD90. An exemplary antigen expressed on a NK cell is selected from the group consisting of CD2, CD8, CD11b, CD16, CD38, CD56, CD57, ADAM17, KIR, KAR, KLR and CD137. An exemplary antigen on a monocyte is selected from the group consisting of CD74, HLA-DR alpha chain, CD14, CD16, CD64 and CD89. An exemplary antigen on a neutrophil is selected from the group consisting of CEACAM6, CEACAM8, CD16b, CD32a, CD89, CD177, CD11a, CD11b, and SLC44A2. The antigen on a leukocyte may also be a checkpoint antigen, which may be selected from the group consisting of LSECtin, CTLA4, PD1, PD-L1, LAG3, B7-H3, B7-H4, KIR and TIM3. Preferably, the antigen on a T cell is CD3, CD4 or CD8, and the antigen on a NK cell is CD16 or CD56.
The second binding moiety can bind to an antigen on a diseased cell or disease-associated cell or pathogen. In certain embodiments, the second binding moiety of the antibody can target to an antigen on any type of tumors and any type of tumor cells. An exemplary type of cancer that can be targeted to includes acute lymphoblastic leukemia, acute myelogenous leukemia, biliary cancer, breast cancer, cervical cancer, chronic lymphocytic leukemia, chronic myelogenous leukemia, colorectal cancer, endometrial cancer, esophageal cancer, gastric cancer, head and neck cancer, Hodgkin's lymphoma, lung cancer, bone marrow thyroid cancer, non-Hodgkin's lymphoma, multiple myeloma, kidney cancer, ovarian cancer, pancreatic cancer, glioma, melanoma, liver cancer, prostate cancer, bladder cancer, neuroendocrine cancer, gastrointestinal pancreatic tumor, exocrine pancreatic cancer and Ewing sarcoma. However, a skilled artisan in the art will recognize that a known tumor-associated antigen is actually useful for any type of cancer.

The antigen on a tumor cell or cancer cell can be selected from the group consisting of: carbonic anhydrase IX, alpha-fetoprotein, alpha-actinin-4, A3, A33 antibody-specific antigen, AFP, ART-4, B7, Ba 733, BAGE, BrE3-antigen, CA125, CAMEL, CAP-1, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD79b, CD80, CD83, CD95, CD126, CD132, CD133, CD138, CD147, CD154, CDC27, CDK-4/m, CDKN2A, CXCR4, CXCR7, CXCL12, HIF-1alpha, colon-specific antigen-p (CSAp), CEA(CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, gp100, GROB, HLA-DR, HM1.24, human chorionic gonadotropin (HCG) and its subunit, HER2/neu, HMGB-1, hypoxia-inducible factor (HIF-1), HSP70-2M, HST-2, Ia, IGF-1R, IFN-$\gamma$, IFN-$\alpha$, IFN-$\beta$, IL-2, IL-4R, IL-6R, IL-13R, IL-15R, IL-17R, IL-18R, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, pancreatic cancer mucin, placental growth factor, p53, PLAGL2, prostate acid phosphatase, PSA, PRAME, PSMA, PIGF, ILGF, ILGF-1R, IL-6, IL-25, RS5, RANTES, T101, SAGE, S100, survivin, survivin-2B, TAC, TAG-72, tenascin, TRAIL receptor, TNF-alpha, Tn antigen, Thomson-Friedrich antigen, tumor necrosis antigen, TROP-2, VEGFR, ED-B fibronectin, WT-1, 17-1A-antigen, complement factor C3, C3a, C3b, C5a, C5, angiogenesis marker, bc1-2, bc1-6, Kras, cMET, and oncogene product.

The antigen on a tumor cell or cancer cell may also a cell surface protein selected from the group consisting of cholecystokinin B Receptor, gonadotropin releasing hormone receptor, somatostatin receptor 2, avb3 integrin, gastrin releasing peptide receptor, neurokinin 1 receptor, melanocortin 1 receptor, neurotensin receptor, neuropeptide Y receptor and C-type lectin-like molecule 1.

The second binding moiety can bind to a cell associated with an inflammatory disease and/or an autoimmune disease. The inflammatory disease and/or autoimmune disease may, for example, be selected from the group consisting of: rheumatoid arthritis, ankylosing spondylitis, psoriasis, psoriatic arthritis, ulcerative colitis, Crohn's disease, systemic lupus erythematosus, juvenile diabetes, autoimmune uveitis, multiple sclerosis, Parkinson's disease, Alzheimer's disease and ischemia-reperfusion injury (see WO2014/180577).

The pathogen may be selected from the group consisting of HIV virus, *Mycobacterium tuberculosis, Streptococcus agalactiae,* meticillin-resistant *Staphylococcus aureus, Legionella pneumophila, Streptococcus pyogenes, Escherichia coli, Neisseria gonorrhoeae, Neisseria meningitidis, Pneumococcus, Cryptococcus neoformans, Histoplasma capsulatum, Hemophilis influenzae B, Treponema pallidum,* Lyme disease spirochetes, *Pseudomonas aeruginosa, Mycobacterium leprae, Brucella abortus,* Rabies virus, Influenza virus, Cytomegalovirus, Type I herpes simplex virus, type II herpes simplex virus, human serum parvo-like virus, respiratory syncytial virus, varicella-zoster virus, hepatitis B virus, hepatitis C virus, measles virus, adenovirus, human T cell leukemia virus, Epstein-Barr virus, murine leukemia virus, mumps virus, vesicular stomatitis virus, Sindbis virus, lymphocyte choriomeningitis virus, Wart virus, Blue tongue virus, Sendai virus, Cat leukemia virus, Reovirus, Poliovirus, Simian virus 40, mouse mammary tumor virus, Dengue fever virus, rubella virus, West Nile virus, *Plasmodium falciparum, Plasmodium vivax, Toxoplasma gondii, Trypanosoma rangeli, Trypanosoma cruzi, Trypanosoma rhodesiensei, Trypanosoma brucei, Schistosoma mansoni, Schistosoma japonicum, Babesia bovis, Elmeria tenella, Onchocerca volvulus, Leishmania tropica, Trichinella spiralis, Theileria parva, Taenia hydatigena, Taenia ovis, Taenia saginata, Echinococcus granulosus, Mesocestoides corti, Mycoplasma arthritidis, M.hyorhinis, M.orale, M. arginini, Acholeplasma laidlawii, M. salivarium* and *M. pneumoniae.*

The antibody used may be a murine antibody, sheep antibody and so on, chimeric antibody, humanized and human antibody or a fragment thereof. The antibody may also be a heavy chain antibody (such as a camelid antibody) or a fragment thereof. The multispecific antibody can be multispecific antibodies of various forms, such as a single-chain variable fragment (scFv)-derived form, a form comprising an antibody heavy chain variable region fragment, a form based on immunoglobulin G (IgG) as well as combinations of the above forms. The antibody may comprise an scFv and/or an antibody heavy chain variable region fragment.

The nanomaterial may be a pharmaceutically acceptable nanomaterial, preferably a biodegradable nanomaterial, such as a nanoparticle. More preferably, the nanomaterial is any one of or a mixture of at least two of poly(lactic acid-co-glycolic acid), polylactic acid, polycaprolactone, polybutylene succinate, polyaniline, polycarbonate, poly(glycolide-co-lactide) or poly(glycolide-co-caprolactone), most preferably poly(lactic acid-co-glycolic acid) (PLGA), polylactic acid (PLA) and/or polycaprolactone (PCL). The average particle size of the nanoparticle may be, for example, about 10-990 nm, for example, about 900 nm, about 850 nm, about 800 nm, about 750 nm, about 700 nm, about 650 nm, about 600 nm, about 550 nm, about 500 nm, about 450 nm, about 400 nm, about 350 nm, about 300 nm, about 250 nm, about 200 nm, about 150 nm, about 100 nm or less, such as about 90 nm, about 80 nm, about 70 nm, about 60 nm, about 50 nm, about 40 nm, about 30 nm, about 20 nm, or about 10 nm. Preferably, the nanoparticle has an average particle size in the range of 10 to 500 nm, more preferably in the range of 10 to 300 nm.

The present disclosure also provides a pharmaceutical composition comprising the multispecific antibody conjugate disclosed herein. The pharmaceutical composition may also comprise a leukocyte, such as one that binds to the multispecific antibody conjugate. The leukocyte preferably is a T cell and/or NK cell. The pharmaceutical composition may also comprise other therapeutic agent(s) for treating the disease.

The present disclosure also provides a method of treating a disease or condition comprising administering the conjugate or the pharmaceutical composition to a subject in need thereof. The method of treatment may further comprise sequential or simultaneous administration of other therapeutic agent(s) for treating the disease.

The present disclosure also provides the use of the conjugate and the pharmaceutical composition in the manufacture of a medicament for treating or preventing the disease or condition.

The present disclosure also relates to the conjugate and the pharmaceutical composition for use in treating or preventing the disease or condition.

In certain embodiments, the conjugate and the pharmaceutical composition disclosed herein can be used to treat cancer, such as the cancer disclosed herein. An exemplary type of cancer includes acute lymphoblastic leukemia, acute myelogenous leukemia, biliary cancer, breast cancer, cervical cancer, chronic lymphocytic leukemia, chronic myelogenous leukemia, colorectal cancer, endometrial cancer, esophageal cancer, gastric cancer, head and neck cancer, Hodgkin's lymphoma, lung cancer, bone marrow thyroid cancer, non-Hodgkin's lymphoma, multiple myeloma, kidney cancer, ovarian cancer, pancreatic cancer, glioma, melanoma, liver cancer, prostate cancer, bladder cancer, neuroendocrine cancer, gastrointestinal pancreatic tumor, exocrine pancreatic cancer and Ewing sarcoma.

In other embodiments, a subject infected with a pathogenic organism such as a bacterium, virus, or fungus can be treated using the conjugate or the pharmaceutical composition disclosed herein. Exemplary fungus that can be treated includes Microsporum, Trichophyton, Epidermophyton, Sporothrix schenckii, Cryptococcus neoformans, Coccidioides immitis, Histoplasma capsulatum, Blastomyces dermatitidis or Candida albicans. Exemplary virus includes human immunodeficiency virus (HIV), herpes virus, cytomegalovirus, rabies virus, influenza virus, human papilloma virus, hepatitis B virus, hepatitis C virus, Sendai virus, Cat leukemia virus, Reovirus, poliovirus, human serum parvo-like virus, Simian virus 40, respiratory syncytial virus, mouse mammary tumor virus, varicella-zoster virus, dengue virus, rubella virus, measles virus, adenovirus, human T cell leukemia virus, Epstein-Barr virus, murine leukemia virus, mumps virus, vesicular stomatitis virus, Sindbis virus, lymphocyte choriomeningitis virus or Blue tongue virus. Exemplary bacterium includes Bacillus anthracis, Streptococcus agalactiae, Legionella pneumophila, Streptococcus pyogenes, Escherichia coli, Neisseria gonorrhoeae, Neisseria meningitidis, Pneumococcus, Hemophilis influenzae B, Treponema pallidum, Lyme disease spirochetes, Pseudomonas aeruginosa, Mycobacterium leprae, Brucella abortus, Mycobacterium tuberculosis or Mycoplasma.

Specifically, the following inventions are disclosed herein:

1. An antibody conjugate comprising a multispecific antibody conjugated to a nanomaterial, preferably a biodegradable nanomaterial, such as a nanoparticle, preferably the antibody binding to both a cytotoxic effector cell and a target cell.

2. The antibody conjugate according to item 1, wherein the antibody comprises a binding moiety that specifically binds to a cytotoxic effector cell.

3. The antibody conjugate according to item 2, wherein the cytotoxic effector cell comprises a T cell.

4. The antibody conjugate according to item 3, wherein the antibody comprises a binding moiety that specifically binds to an antigen selected from the group consisting of:

CD2, CD3, CD4, CD5, CD6, CD8, CD25, CD28, CD30, CD40, CD40L, CD44, CD45, CD69 and CD90, and preferably the antigen is CD3.

5. The antibody conjugate according to item 2, wherein the cytotoxic effector cell comprises a NK cell.

6. The antibody conjugate according to item 5, wherein the antibody comprises a binding moiety that specifically binds to an antigen selected from the group consisting of:

CD2, CD8, CD11b, CD16, CD38, CD56, CD57, ADAM17, KIR, KAR, KLR and CD137, and preferably the antigen is selected from the group consisting of CD16 and CD56.

7. The antibody conjugate according to any of the preceding items, wherein the antibody comprises a binding moiety that specifically binds to a target cell, preferably a disease-associated cell.

8. The antibody conjugate according to item 7, wherein the disease is a tumor or a cancer.

9. The antibody conjugate according to item 8, wherein the tumor or cancer is selected from the group consisting of: non-Hodgkin's lymphoma, B cell lymphoma, B cell leukemia, T cell lymphoma, T cell leukemia, acute lymphocytic leukemia, chronic lymphocytic leukemia, Burkitt's lymphoma, Hodgkin's lymphoma, hairy cell leukemia, acute myelogenous leukemia, chronic myelogenous leukemia, multiple myeloma, glioma, Waldenstrom's macroglobulinemia, carcinoma, melanoma, sarcoma, glioma, skin cancer, oral cancer, gastrointestinal cancer, colon cancer, stomach cancer, lung cancer, lung carcinoma, breast cancer, ovarian cancer, prostate cancer, uterine cancer, endometrial cancer, cervical cancer, bladder cancer, pancreatic cancer, bone cancer, liver cancer, gallbladder cancer, kidney cancer, testicular cancer, epithelial cancer, colorectal cancer, gastric cancer, brain cancer, glioblastoma, pancreatic cancer, myeloid leukemia, cervical cancer, medullary thyroid carcinoma, astrocytoma, prostate adenocarcinoma, bladder cancer, neuroendocrine cancer, gastrointestinal pancreatic tumor, exocrine pancreatic cancer and Ewing sarcoma.

10. The antibody conjugate according to item 9, wherein the antibody comprises a binding moiety that specifically binds to an antigen selected from the group consisting of:

carbonic anhydrase IX, alpha-fetoprotein, alpha-actinin-4, A3, A33 antibody-specific antigen, AFP, ART-4, B7, Ba 733, BAGE, BrE3-antigen, CA125, CAMEL, CAP-1, CASP-8/m, CCCL19, CCCL21, CD1, CD1a, CD2, CD3, CD4, CD5, CD8, CD11A, CD14, CD15, CD16, CD18, CD19, CD20, CD21, CD22, CD23, CD25, CD29, CD30, CD32b, CD33, CD37, CD38, CD40, CD40L, CD45, CD46, CD52, CD54, CD55, CD59, CD64, CD66a-e, CD67, CD70, CD70L, CD74, CD79a, CD79b, CD80, CD83, CD95, CD126, CD132, CD133, CD138, CD147, CD154, CDC27, CDK-4/m, CDKN2A, CXCR4, CXCR7, CXCL12, HIF-1alpha, colon-specific antigen-p (CSAp), CEA(CEACAM5), CEACAM6, c-met, DAM, EGFR, EGFRvIII, EGP-1, EGP-2, ELF2-M, Ep-CAM, Flt-1, Flt-3, folate receptor, G250 antigen, GAGE, gp100, GROB, HLA-DR, HM1.24, human chorionic gonadotropin (HCG) and its subunit, HER2/neu, HMGB-1, hypoxia-inducible factor (HIF-1), HSP70-2M, HST-2, Ia, IGF-1R, IFN-$\gamma$, IFN-$\alpha$, IFN-$\beta$, IL-2, IL-4R, IL-6R, IL-13R, IL-15R, IL-17R, IL-18R, IL-6, IL-8, IL-12, IL-15, IL-17, IL-18, IL-23, IL-25, insulin-like growth factor-1 (IGF-1), KC4-antigen, KS-1-antigen, KS1-4, Le-Y, LDR/FUT, macrophage migration inhibitory factor (MIF), MAGE, MAGE-3, MART-1, MART-2, NY-ESO-1, TRAG-3, mCRP, MCP-1, MIP-1A, MIP-1B, MIF, MUC1, MUC2, MUC3, MUC4, MUC5ac, MUC13, MUC16, MUM-1/2, MUM-3, NCA66, NCA95, NCA90, pancreatic cancer mucin, placental growth factor, p53, PLAGL2, prostate acid phosphatase, PSA, PRAME, PSMA, PIGF, ILGF, ILGF-1R, IL-6, IL-25, RS5, RANTES, T101, SAGE, S100, survivin, survivin-2B, TAC, TAG-72, tenascin, TRAIL receptor, TNF-alpha, Tn antigen, Thomson-Friedrich antigen, tumor necrosis antigen, TROP-2, VEGFR, ED-B fibronectin, WT-1, 17-1A-antigen, complement factor C3, C3a, C3b, C5a, C5, angiogenesis marker, bc1-2, bc1-6, Kras, cMET, and oncogene product, and preferably the antigen is selected from the group consisting of MUC1, CEA, CD19 and CD20.

11. The antibody conjugate according to item 7, wherein the target cell is a cell associated with an inflammatory disease and/or an autoimmune disease.

12. The antibody conjugate according to item 11, wherein the inflammatory disease and/or autoimmune disease is selected from the group consisting of: rheumatoid arthritis, ankylosing spondylitis, psoriasis, psoriatic arthritis, ulcerative colitis, Crohn's disease, systemic lupus erythematosus, juvenile diabetes, autoimmune uveitis, multiple sclerosis, Parkinson's disease, Alzheimer's disease and ischemia-reperfusion injury.

13. The antibody conjugate according to any of items 1 to 12, wherein the antibody is selected from the group consisting of a single-chain variable fragment (scFv)-derived form, a form comprising an antibody heavy chain variable region fragment, a form based on immunoglobulin G (IgG) as well as combinations of the above forms.

14. The antibody conjugate according to item 13, wherein the antibody comprises a single chain variable region fragment (scFv) and/or an antibody heavy chain variable region fragment.

15. The antibody conjugate according to any of the preceding items, wherein the antibody is a bispecific antibody or a trispecific antibody, preferably a bispecific antibody.

16. The antibody conjugate according to item 15, wherein the bispecific antibody is selected from the group consisting of a diabody, a tandem diabody, BiTe (a bispecific T cell adaptor), DART (double affinity re-targeting), Triomab, and DVD-Ig (double variable domain antibody).

17. The antibody conjugate according to item 15, wherein the antibody is in the form of an antibody selected from the group consisting of a single-chain diabody (scDb), a tandem scDb (Tandab), a linear dimeric scDb (LD-scDb), a circular dimeric scDb (CD-scDb), a tandem di-scFv, a tandem tri-scFv, a tri(a)body, a bispecific Fab2, a di-miniantibody, a tetrabody, an scFv-Fc-scFv fusion, a single domain antigen-binding fragment fused with Fab (S-Fab), a tandem di-VHH, a di-diabody, a DVD-Ig, an IgG-scFab, an scFab-dsscFv, a Fv2-Fc, an IgG-scFv fusion, a knob-in-hole (KiH), DNL complexes and DuoBodies, and preferably the IgG-scFv fusion is a bsAb, a Bs1Ab, a Bs2Ab, a Bs3Ab, a TslAb or a Ts2Ab.

18. The antibody conjugate according to item 17, wherein the antibody is a bispecific T cell adaptor.

19. The antibody conjugate according to item 18, wherein the antibody is a bispecific T cell adaptor that specifically binds to CD3 and specifically binds to CD19, preferably blinatumomab.

20. The antibody conjugate according to item 18, wherein the antibody is a tandem di-scFv, an S-Fab or a tandem di-VHH, preferably a tandem di-VHH comprising the sequences as set forth in SEQ ID NOs: 1 and 2.

21. The antibody conjugate of any of items 13-17, wherein the antibody comprises a binding moiety that specifically binds to a NK cell.

22. The antibody conjugate according to item 21, wherein the antibody is a bispecific antibody that binds to both CD 16 and CEA.

23. The antibody conjugate according to the item 21 or 22, wherein the antibody is a tandem di-scFv.

24. The antibody conjugate according to any of the preceding items, wherein the nanomaterial is any one of or a mixture of at least two of poly(lactic acid-co-glycolic acid), polylactic acid, polycaprolactone, polybutylene succinate, polyaniline, polycarbonate, poly(glycolide-co-lactide) and poly(glycolide-co-caprolactone), most preferably poly(lactic acid-co-glycolic acid) (PLGA), polylactic acid (PLA) and/or polycaprolactone (PCL).

25. The antibody conjugate according to item 24, wherein the nanoparticle has an average diameter in the range of 10-990 nm, preferably in the range of 10 to 500 nm, more preferably in the range of 10 to 300 nm, preferably an average diameter of about 400 nm, about 350 nm, about 300 nm, about 250 nm, about 200 nm, about 150 nm, about 100 nm or less than 100 nm, for example about 90 nm, about 80 nm, about 70 nm, about 60 nm, about 50 nm, about 40 nm, about 30 nm, about 20 nm, or about 10 nm.

26. A pharmaceutical composition comprising the antibody conjugate of any of items 1-25.

27. The pharmaceutical composition according to item 25, which further comprises a cytotoxic effector cell, which is preferably a leukocyte and selected from the group consisting of a T cell, an NK cell, an NKT cell, a macrophage, a neutrophil, and an eosinophil, preferably a cytotoxic effector cell comprising a cell selected from the group consisting of a T cell and an NK cell, preferably the pharmaceutical composition being used for immunotherapy.

28. Use of the antibody conjugate of any of items 1 to 25, or the pharmaceutical composition of item 26 or 27 for the preparation of an immunotherapeutic medicament, which is preferably used for treating a tumor or cancer or for treating an inflammatory disease and/or autoimmune disease.

29. The use according to item 28, wherein the tumor or cancer is selected from the group consisting of:
acute lymphoblastic leukemia, acute myelogenous leukemia, biliary cancer, breast cancer, cervical cancer, chronic lymphocytic leukemia, chronic myelogenous leukemia, colorectal cancer, endometrial cancer, esophageal cancer, gastric cancer, head and neck cancer, Hodgkin's lymphoma, lung cancer, bone marrow thyroid cancer, non-Hodgkin's lymphoma, multiple myeloma, kidney cancer, ovarian cancer, pancreatic cancer, glioma, melanoma, liver cancer, prostate cancer, bladder cancer, neuroendocrine cancer, gastrointestinal pancreatic tumor, exocrine pancreatic cancer and Ewing sarcoma.

30. The use according to item 28, wherein the inflammatory disease and/or autoimmune disease is selected from the group consisting of:
rheumatoid arthritis, ankylosing spondylitis, psoriasis, psoriatic arthritis, ulcerative colitis, Crohn's disease, systemic lupus erythematosus, juvenile diabetes, autoimmune uveitis, multiple sclerosis, Parkinson's disease, Alzheimer's disease and ischemia-reperfusion injury.

31. A method for treating a tumor or cancer or an inflammatory disease and/or autoimmune disease, comprising administering the antibody conjugate of any of items 1 to 25 or the pharmaceutical composition of item 26 or 27.

32. The antibody conjugate, pharmaceutical composition, use or method according to any of the preceding items, wherein the antibody conjugate further comprises other antibody moieties that specifically bind to a cytotoxic effector cell or a target cell.

33. The conjugate, pharmaceutical composition, use or method according to any of the preceding items, wherein the cytotoxic effector cell comprises a cell selected from the group consisting of a T cell, an NK cell, and an NKT cell, and preferably is selected from the group consisting of a T cell, an NK cell, an NKT cell, and a CIK cell.

34. The conjugate, pharmaceutical composition, use or method of any of the preceding items, wherein the antibody comprises a binding moiety that specifically binds to the antigen CD123.

35. The conjugate, pharmaceutical composition, use or method of any of the preceding items, wherein the antibody comprises a binding moiety that specifically binds to the antigen CD89.

## Brief Description of the Drawings

Figure 1 is a schematic view showing the structure of the bispecific antibody BiTE(CD16-MUC1).

Figure 2 shows an electropherogram for the purification of the bispecific antibody BiTE(CD16-MUC1).

Figure 3 is a schematic view showing the structure of the bispecific antibody BiTE(CD16-CEA).

Figure 4 shows the expression and purification of the bispecific antibody BiTE(CD16-CEA). Figure 4A shows the profile of the Ni-NTA purification process; Figure 4B shows the profile of the Q-sepharose HP purification process.

Figure 5 shows the killing rate of the bispecific antibody BiTE(CD16-MUC1) and the antibody conjugate BiTE(CD16-MUC1)-PLGA in combination with NK cells on lung cancer cell A549 (human non-small cell lung cancer cells).

Figure 6 shows the killing rate of the bispecific antibody BiTE(CD16-CEA) and the antibody conjugate BiTE(CD16-CEA)-PLGA in combination with NK cells on intestinal cancer cell HCT116.

Figure 7 shows the killing rate of the bispecific antibody CD16-MUC1 BiTE and the antibody conjugate CD16-MUC1 BiTE+PLGA in combination with NK cells on lung cancer cell A549 (human non-small cell lung cancer cells).

Figure 8 shows the killing rate of the bispecific antibody BiTE(CD3-MUC1) and the antibody conjugate BiTE(CD3-MUC1)-PLGA, after being placed at 37 °C for different time, in combination with NK cells on lung cancer cell A549 (human non-small cell lung cancer cells).

Figure 9 shows the killing rate of the bispecific antibody CD3-CEA BiTE and the antibody conjugate CD3-CEA BiTE+PLGA NPs in combination with T cells on lung cancer cell A549 (human non-small cell lung cancer cells).

Figure 10 shows sequences of target fragments HCBF-6 (NcoI-UCHT1-VH-HindIII-CH-XhoI-CEA-Flag) and HCBF-6 (NcoI-UCHT1-VH-HindIII-CH-XhoI-CEA-Flag) for preparing the bispecific antibody CD3-CEA BiTE (i.e. CEA-S-Fab).

**Description**

Before the method and composition of the present invention are described, it should be understood that the invention is not limited to the specific method or composition described, and thus may, of course, vary. It should also be understood that the terms used herein are only illustrative, but not limiting. The description of the embodiments is provided for guiding a skilled artisan in the art to make and use the present invention, and is not intended to limit the scope of the invention, and also is not intended to indicate that the following experiments are all or only the experiments performed. Efforts have been made to ensure the accuracy of the number used (e.g., amount, temperature, etc.), but some experimental errors and deviations should be considered.

Unless defined otherwise, all technical and scientific terms used herein have the same meaning as commonly understood by a skilled artisan in the art. Although any method and material similar or equivalent to those described herein can be used in the practice or testing of the present invention, some potential and preferred methods and materials are now described. All publications mentioned herein are hereby incorporated by reference to disclose and describe the methods and/or materials related to the cited publications. It will be understood that in the event of a conflict, the present disclosure will control.

As will be apparent to a skilled artisan in the art upon reading this disclosure, each individual embodiment described and illustrated herein has discrete components and features that can be readily separated from or combined with the features in several other embodiments, without departing the scope or spirit of the invention. Any of the enumerated methods can be implemented in the order of the recited events or in any other order that is logically possible.

Unless stated otherwise, the article "a" or "an" as used in the description and in the appended claims means "one or more".

Where a range of values is provided, it should be understood that each intermediate value (up to one tenth of the lower limit unit) between the upper and lower limits of the range is also specifically disclosed, unless the context clearly dictates otherwise. The various smaller ranges between any such values or intermediate values within the range as well as other such values or intermediate values within the range are included in the invention. The upper and lower limits of these smaller ranges may be independently included in or excluded from the range and are subject to any particular exclusion in the range, wherein either or both or none of the upper and lower limits included in each range within the smaller range is also included in the present invention. Where the stated range includes one or both of the limits, the scope of the exclusion of either or both of such limits is also included in the invention.

As used herein, an "antibody" refers to a full-length (i.e., naturally occurring or formed by the process of rearrangement of a normal immunoglobulin gene segment) immunoglobulin molecule (e.g., an IgG antibody) or an immunoactive (i.e., specific binding) part of such immunoglobulin molecule (e.g., an antibody fragment). An "antibody" includes a monoclonal antibody, a polyclonal antibody, a bispecific antibody, a multispecific antibody, a murine antibody, a heavy chain antibody (such as a camelid antibody), a chimeric antibody, a humanized antibody and a human antibody, and the like. A "heavy chain antibody" is an antibody that contains only a heavy chain and does not contain a light chain, such as a camelid antibody, a cartilaginous fish (such as a shark) antibody, and the like.

An "antibody fragment" is a part of an intact antibody, such as $F(ab')_2$, $F(ab)_2$, Fab', Fab, Fv, scFv, dAb, and the like. Regardless of the structure, the antibody fragment binds to the same antigen as recognized by the full length antibody. For example, an antibody fragment comprises an isolated fragment consisting of variable regions, such as an "Fv" fragment consisting of a heavy or light chain variable region or a recombinant single-chain polypeptide molecule ("scFv protein") in which the light chain and heavy chain variable regions are joined by a peptide linker. A "single-chain antibody" is often abbreviated as "scFv" and consists of a polypeptide chain comprising $V_H$ and $V_L$ domains that interact with each other to form an antigen-binding site. The $V_H$ and $V_L$ domains are typically joined by a peptide having from 1 to 25 amino acid residues. An antibody fragment also includes a bifunctional antibody, a trifunctional antibody, and a single-domain antibody (dAb). An "antibody fragment" also includes a fragment of the heavy chain antibody, such as a single-domain antibody (sdAb) or a fragment comprising a variable region.

A "multispecific antibody" is an antibody that can simultaneously bind to at least two targets with different structures (e.g., two different antigens, two different epitopes on the same antigen). A "bispecific antibody" is an antibody that can simultaneously bind to two targets with different structures. The bispecific antibody or multispecific antibody used may be a known type of antibody. For example, the multispecific antibody (e.g., a bispecific antibody) can be a single-chain variable fragment (scFv)-derived form, a form comprising an antibody heavy chain variable region fragment, a form based on immunoglobulin G (IgG) as well as combinations of the above forms. The multispecific antibodies (e.g., bispecific antibodies) can be a diabody, a tandem diabody, BiTe (a bispecific T cell adaptor), DART (double affinity retargeting), Triomab, and DVD-Ig (double variable domain antibody); can be a single-chain diabody (scDb, e.g., a bispecific monomer scDb), a tandem scDb (Tandab), a linear dimeric scDb (LD-scDb), a circular dimeric scDb (CD-scDb), a tandem di-scFv (e.g., bispecific T cell adaptor (BiTE)), a tandem tri-scFv, a tri(a)body, a bispecific Fab2, a di-miniantibody, a tetrabody, an scFv-Fc-scFv fusion, a single domain antigen-binding fragment fused with Fab (S-Fab) (see such as CN106188305A), a di-diabody, a DVD-Ig, an IgG-scFab, an scFab-dsscFv, a Fv2-Fc, an IgG-scFv fusion, a knob-in-hole (KiH), Dock and Lock (DNL) complexes (see such as CN101534865A) and DuoBodies; and the IgG-scFv fusion can be a bsAb, a Bs1Ab, a Bs2Ab, a Bs3Ab, a TslAb or a Ts2Ab. The multispecific antibody can also be a multispecific antibody, such as a bispecific antibody, comprising two or more antibody heavy chain variable regions.

The multispecific antibody can comprise a first binding moiety binding to a leukocyte and a second binding moiety binding to an antigen on a cell associated with a disease or a cell to be cleared (a target cell). The first binding moiety and/or the second binding moiety can have one or more binding specificities.

An exemplary antigen on a T cell includes CD2, CD3, CD4, CD5, CD6, CD8, CD25, CD28, CD30, CD40, CD40L, CD44, CD45, CD69 and CD90. For a NK cell, other exemplary antigen can be selected from the group consisting of CD8, CD16, CD56, CD57, ADAM17 and CD137; for a monocyte, an exemplary antigen can be selected from the group consisting of CD74, HLA-DR alpha chain, CD14, CD16, CD64 and CD89; and for a neutrophil, an exemplary antigen includes CEACAM6, CEACAM8, CD16b, CD32a, CD89, CD177, CD11a, CD11b and SLC44A2. In a preferred embodiment, the first binding moiety for NK binds to CD16 or CD56. As discussed below, many examples of the disease-associated antigen, such as a tumor-associated antigen (TAA) or an antigen expressed by a pathogen, are known. Preferably, the TAA is MUC1, CD19, CD20, CD33, CD38, EGFR and HER2.

In certain embodiments, the antigen on the target cell can be a cancer cell receptor or a cancer associated antigen, particularly selected from the group consisting of B cell line antigens (CD19, CD20, CD21, CD22, CD23, etc.), VEGF, VEGFR, EGFR, carcinoembryonic antigen (CEA), placental growth factor (PIGF), tenascin, *HER-2/neu,* EGP-1, EGP-2, CD25, CD30, CD33, CD38, CD40, CD45, CD52, CD74, CD80, CD138, NCA66, CEACAM1, CEACAM6 (carcinoembryonic antigen-associated cell adhesion molecule 6), MUC1, MUC2, MUC3, MUC4, MUC16, IL-6, alpha-fetoprotein (AFP), A3, CA125, colon-specific antigen-p (CSAp), folate receptor, HLA-DR, human chorionic gonadotropin (HCG), Ia, EL-2, insulin-like growth factor (IGF) and IGF receptor, KS-1, Le(y), MAGE, necrotic antigen, PAM-4, prostatic acid phosphatase (PAP), Prl, prostate specific antigen (PSA), prostate specific membrane antigen (PSMA), S100, T101, TAC, TAG72, TRAIL receptor and carbonic anhydrase IX.

In certain embodiments, the target cell can also be a cell associated with an inflammatory disease and/or an autoimmune disease. The inflammatory disease and/or autoimmune disease can, for example, be selected from the group consisting of rheumatoid arthritis, ankylosing spondylitis, psoriasis, psoriatic arthritis, ulcerative colitis, Crohn's disease, systemic lupus erythematosus, juvenile diabetes, autoimmune uveitis, multiple sclerosis, Parkinson's disease, Alzheimer's disease and ischemia-reperfusion injury.

The target cell can also be a pathogen or a cell infected with a pathogen. The pathogen can be, for example, a pathogenic organism such as a bacterium, a virus or a fungus.

The binding moiety useful in the claimed antibody conjugate is preferably an antibody or a fragment thereof. Techniques for preparing a monoclonal antibody that are actually directed against any target antigen are well known in the art. Known and/or commercially available antibodies can also be used. For example, an antibody that may be used is selected from the group consisting of hR1 (anti-IGF-1R), hPAM4 (anti-mucin), KC4 (anti-mucin), hA20 (anti-CD20), hA19 (anti-CD19), hIMMU31 (anti-AFP), hLL1 (anti-CD74), hLL2 (anti-CD22), RFB4 (anti-CD22), hMu-9 (anti-CSAp), hL243 (anti-HLA-DR), hMN-14 (anti-CEACAM5), hMN-15 (anti-CEACAM6), hRS7 (anti-TROP-2), hMN-3 (anti-CEACAM6), CC49 (anti-TAG-72), J591 (anti-PSMA), D2/B (anti-PSMA), G250 (anti-carbonic anhydrase IX), infliximab (anti-TNF-a), certolizumab pegol (anti-TNF-a), adalimumab (anti-TNF-a), alemtuzumab (anti-CD52), bevacizumab (anti-VEGF), cetuximab (anti-EGFR), gemtuzumab (anti-CD33), ibritumomab (anti-CD20), panitumumab (anti-EGFR), rituximab (anti-CD20), tositumomab (anti-CD20), GA101 (anti-CD20), trastuzumab (anti-HER2/neu), pertuzumab, tocilizumab (anti-IL-6 receptor), basiliximab (anti-CD25), daclizumab (anti-CD25), efalizumab (anti-CD11a), muromonab-CD3 (anti-CD3 receptor), natalizumab (anti-α4 integrin), BWA-3 (anti-histone H2A/H4), LG2-1 (anti-histone H3), MRA12 (anti-histone HI), PR1-1 (anti-histone H2B), LG11-2 (anti-histone H2B), LG2-2 (anti-histone H2B), P4/D10 (anti-gp120) and omalizumab (anti-IgE). Fragments of the above antibodies can be used.

Exemplary antibodies for the treatment of, for example, cancer include, but are not limited to, LL1 (anti-CD74), LL2 or RFB4 (anti-CD22), veltuzumab (hA20, anti-CD20), rituximab (anti-CD20), obinutuzumab (GA101, anti-CD20), ranibizumab (anti-PD1), nivolumab (anti-PD1), MK-3475 (anti-PD1), AMP-224 (anti-PD1), pidilizumab (anti-PD1), MDX-1105 (anti-PD-L1), MEDI4736 (anti-PD-LI), MPDL3280A (anti-PD-LI), BMS-936559 (anti-PD-L1), Ipilimumab (anti-CTLA4), Trevizumab (anti-CTL4A), RS7 (anti-epithelium glycoprotein-1 (EGP-1, also known as TROP-2)), PAM4 or KC4 (both as anti-mucin), MN-14 (anti-carcinoembryonic antigen (CEA, also known as CD66e or CEACAM5)), MN-15 or MN-3 (anti-CEACAM6), Mu-9 (anti-colon specific antigen-p), Immu 31 (anti-alphafetoprotein), R1 (anti-IGF-1R), A19 (anti-CD19), TAG-72 (e.g. CC49), Tn, J591 or HuJ591 (anti-PSMA (prostate specific membrane antigen)), AB-PG1-XG1-026 (anti-PSMA dimer), D2/B (anti-PSMA), G250 (anti-carbonic anhydrase IX MAb), L243 (anti-HLA-DR), alemtuzumab (anti-CD52), bevacizumab (anti-VEGF), cetuximab (anti-EGFR), gemtuzumab (anti-CD33), ibritumomab (anti-CD20), panitumumab (anti-EGFR), tositumomab (anti-CD20), PAM4 (aka krivuzumab, anti-mucin), BWA-3 (anti-histone H2A/H4), LG2-1 (anti-histone H3), MRA12 (anti-histone HI), PR1-1 (anti-histone H2B), LG11-2 (anti-histone H2B), LG2-2 (anti-histone H2B) and trastuzumab (anti-ErbB2). These antibodies are known in the art.

Nanomaterial

The nanomaterial for conjugating the first binding moiety (e.g., an antibody or a fragment thereof) and the second binding moiety (e.g., an antibody or a fragment thereof) may be a pharmaceutically acceptable nanomaterial, preferably a biodegradable nanomaterial, more preferably any one of or a mixture of at least two of poly(lactic acid-co-glycolic acid), polylactic acid, polycaprolactone, polybutylene succinate, polyaniline, polycarbonate, poly(glycolide-co-lactide) or poly(glycolide-co-caprolactone), most preferably poly(lactic acid-co-glycolic acid) (PLGA), polylactic acid (PLA) and/or polycaprolactone (PCL). These nanomaterials and methods for preparing them are known in the art. For example, the nanomaterials can be prepared using the methods described below.

**Preparation of the antibody conjugate of the invention**

The method for preparing the multispecific antibody conjugate of the present invention is described below by taking a bispecific antibody conjugate comprising two antibodies conjugated to a nanoparticle as an example. The preparation method comprises the following steps:

(1) preparing, collecting and activating the nanomaterial;
(2) ligating the nanomaterial obtained in step (1) to a mixture of the first antibody moiety and the second antibody moiety.

In the step (1), the preparation of the nanomaterial comprises: completely dissolving the nanomaterial with a solvent, stirring, adding water to form a uniform emulsion. The agitation may be carried out at a rotation speed of 500 to 20,000 rpm/min, for example, the rotation speed may be 500 rpm/min, 700 rpm/min, 800 rpm/min, 1000 rpm/min, 1100 rpm/min, 1200 rpm/min, 1300 rpm/min, 1400 rpm/min, 1480 rpm/min, 1500 rpm/min, 2000 rpm/min, 2200 rpm/min, 2500 rpm/min, 3000 rpm/min, 3500 rpm/min, 4000 rpm/min, 4200 rpm/min, 4500 rpm/min, 5000 rpm/min, 5500 rpm/min, 6000 rpm/min, 6500 rpm/min, 7000 rpm/min, 7500 rpm/min, 8000 rpm/min, 8500 rpm/min, 9000 rpm/min, 9500 rpm/min, 10000 rpm/min, 11000 rpm/min, 12000 rpm/min, 13000 rpm/min, 14000 rpm/min, 15000 rpm/min, 16000 rpm/min, 17000 rpm/min, 18000 rpm/min, 19000 rpm/min or 20000 rpm/min. Higher speed can be used if necessary.

Preferably, the nanomaterial is any one of or a mixture of at least two of poly(lactic acid-co-glycolic acid) (PLGA), polylactic acid (PLA), polycaprolactone (PCL), polybutylene succinate, polyaniline, polycarbonate, poly(glycolide-co-lactide) or poly(glycolide-co-caprolactone).

Preferably, the solvent is any one of or a mixture of at least two of acetone, butanone, methanol, ethanol or isopropanol.

Preferably, the collection of the nanomaterial comprises: collecting the prepared nanomaterial by centrifugation, and then washing the nanomaterial by resuspending in deionized water twice. The centrifugation can be carried out at a rotation speed of 8000-15000 rpm/min, for example, the rotation speed can be 8000 rpm/min, 9000 rpm/min, 10000 rpm/min, 11000 rpm/min, 12000 rpm/min, 13000 rpm/min, 14000 rpm/min, 14500 rpm/min. , 14800 rpm/min, 15000 rpm/min. Higher speeds can be used if necessary. Nanomaterials (nanoparticles) can be collected or further purified by other methods. The nanoparticles may have an average particle size as described above.

Preferably, the activation of the nanomaterial comprises: activating the nanomaterial for 0.5 to 5 hours by using a mixed solvent of 1 to 10 mg/mL 1-ethyl-(3-dimethylaminopropyl)carbodiimide hydrochloride (EDS) and N-hydroxysuccinimide (NHS) at room temperature.

In the step (2) of the invention, the ligation comprises: collecting the activated nanomaterial by centrifugation, and then washing the nanomaterial once with the ligation reaction solution, adding the bispecific antibody to be ligated (and optionally other antibody moiety/moieties mixed in equal amounts) into the ligation reaction solution, and then resuspending the nanomaterial with the ligation reaction solution containing the bispecific antibody (and optionally other antibody moiety/moieties) and conducting the ligation reaction for 0.5 to 5 hours at room temperature. After the reaction, the nanomaterial is collected by centrifugation. The nanomaterial is washed twice in Dulbecco's phosphate buffer saline (D-PBS), and then resuspended in D-PBS and stored at 4 °C. Other methods of activating the nanomaterial can be employed.

The method for preparing the antibody conjugate of the present invention specifically includes the following steps:

(1) preparing a nanomaterial: completely dissolving the nanomaterial in acetone at a concentration of 5 to 30 mg/mL, and adding the solution of the nanomaterial in acetone to the deionized water in 1:4 v/v of acetone and deionized water with magnetic stirring at 500 to 1500 rpm/min, to form a uniform emulsion, and then continuing to stir until the volatilization of acetone;

(2) collecting the nanomaterial: collecting the prepared nanomaterial by centrifugation at 8000 to 15000 rpm/min, and then resuspending in deionized water, which processes are repeated twice for washing the nanomaterial; the nanomaterial may be further purified to obtain a nanomaterial of appropriate size;

(3) activating the nanomaterial: activating the nanomaterial by using a mixed solvent of 1 to 10 mg/mL 1-ethyl-(3-dimethylaminopropyl)carbodiimide hydrochloride and N-hydroxysuccinimide at room temperature for 0.5 to 5 hours;

(4) ligating the nanomaterial to antibodies: collecting the activated nanomaterial by centrifugation, and then washing the nanomaterial once with 0.1 M D-PBS at pH = 8.0, and adding the bispecific antibody to be ligated (and optionally other antibody moiety/moieties mixed in equal amounts) into the ligation reaction solution, and then resuspending the nanomaterial with the ligation reaction solution containing the bispecific antibody (and optionally other antibody moiety/moieties) and conducting the ligation reaction for 0.5 to 5 hours at room temperature. After the reaction, the nanomaterial is collected by centrifugation. The nanomaterial is washed twice in D-PBS, and then resuspended in D-PBS and stored at 4 °C.

## Pharmaceutical composition

The invention also relates to a pharmaceutical composition comprising the antibody conjugate. The pharmaceutical composition may also comprise a leukocyte, such as T cell, NK cell.

The pharmaceutical composition herein may be formulated using one or more physiologically acceptable carriers comprising excipients and auxiliaries which facilitate the entry of the active agent into the pharmaceutically acceptable formulation. Proper formulation depends on the route of administration chosen. For example, an overview of pharmaceutical composition can be found, for example, in Ansel et al, PHARMACEUTICAL DOSAGE FORMS AND DRUG DELIVERY SYSTEMS, 5th edition (Lea & Febiger 1990); and Gennaro (ed.), REMINGTON'S PHARMACEUTICAL SCIENCES, 18th edition (Mack Publishing Company 1990) and its revised edition. The pharmaceutical composition disclosed herein may further comprise one or more diluents, excipients or carriers that are pharmaceutically acceptable. The pharmaceutical composition may comprise a drug or pharmaceutical agent, a carrier, an adjuvant such as a preservative, a stabilizer, a wetting or emulsifying agent, a dissolution promoter, a salt for regulating the osmotic pressure, and/or a buffer.

In addition, the pharmaceutical composition may also contain other therapeutically valuable substances, such as one or more other therapeutic agents for treating the disease. For example, other therapeutic agents may be other agents for treating cancer, such as cyclophosphamide, etoposide, carmustine, vincristine, etc., and may also be antibodies used to treat cancers, antibody-drug conjugates (ADC), interferons and/or checkpoint inhibitor antibodies.

## Method for therapeutic treatment

Various embodiments are directed to methods of treating a disease or condition (e.g., cancer) in a subject, such as a mammal, including a human, domesticated or companion animals, such as a dog and a cat, comprising administering to the subject a therapeutically effective amount of the conjugate or pharmaceutical composition described herein.

The antibody conjugate or pharmaceutical composition described herein may be formulated to be administered subcutaneously or even by other parenteral routes, such as intravenously, intramuscularly, intraperitoneally or intravascularly, to a mammal via, for example, bolus injection or continuous infusion for intravenous administration. Preferably, the antibody conjugate or pharmaceutical composition is infused for a period of less than about 4 hours, and more preferably for a period of less than about 3 hours. For example, the first bolus can be infused over 30 minutes, preferably even 15 minutes, and the remainder is infused over the next 2 to 3 hours. The injectable preparation may be provided in unit dosage form, for example, in ampoules or in multi-dose containers, with a preservative added. The composition may take such forms as a suspension, solution or emulsion in oily or aqueous vehicle, and may contain a formulating agent such as suspending, stabilizing and/or dispersing agents. Alternatively, the active ingredient may be in the form of a powder which is reconstituted with a suitable vehicle, such as sterile pyrogen-free water, before use.

Additional pharmaceutical methods can be employed to control the duration of action of the therapeutic antibody conjugate, pharmaceutical composition, and/or other therapeutic agents. Controlled release formulation can be prepared by using a polymer to complex or adsorb the agent to be administered. For example, a biocompatible polymer includes poly(ethylene-vinyl acetate) copolymer matrix and polyanhydride copolymer matrix of stearic acid dimers and sebacic acid. Sherwood et al, Bio/Technology 10: 1446 (1992). The rate of release from such a matrix depends on the molecular weight of the therapeutic agent, the amount of agent within the matrix, and the size of the dispersed particles. Saltzman et al, Biophys. J. 55: 163 (1989); Sherwood et al., supra. Other solid dosage forms are described in Ansel et al, PHARMACEUTICAL DOSAGE FORMS AND DRUG DELIVERY SYSTEMS, 5th edition (Lea & Febiger 1990); and Gennaro (ed.), REMINGTON'S PHARMACEUTICAL SCIENCES, 18th edition (Mack Publishing Company 1990) and its revised version.

More generally, the dosage of the antibody conjugate administered to a human will vary depending on factors such as the age, weight, height, sex, general medical condition, and past medical history of the patient. It may be desirable to provide the recipient with an antibody conjugate dose as a single intravenous infusion in the range of about 0.1 μg/kg to 25 mg/kg, by the total amount of the first binding moiety and the second binding moiety in the multispecific antibody conjugate, but lower or higher doses can also be administered, as the case may be. For example, for a 70 kg patient, a dose of 0.1 μg/kg -20 mg/kg is 0.7 μg - 1400 mg. The dose can be repeated as needed, for example, once a week for 4 to 10 weeks, once a week for 8 weeks or once a week for 4 weeks. In maintenance therapy, it can also be administered at a lower frequency, if desired, such as every other week for several months, or monthly or quarterly for many months. It is also possible to administer 2, 3, 4, 5 or 6 times continuously for each course of treatment, for example, at an interval of about 10 days, 15 days, 20 days, 25 days, 30 days, 35 days, 40 days, 45 days, 50, 55 days or 60 days or more after continuous administration for the next course of treatment.

The antibody conjugate can be administered with an effector cell such as leukocyte (such as T cell, NK cell), for example, via intravenous reinfusion. When the antibody conjugate is administered with the effector cell, for example, it is performed once a week for 4-10 weeks, once weekly for 8 weeks or once weekly for 4 weeks. In maintenance therapy, the antibody conjugate and effector cell can also be administered at a lower frequency if desired, such as every other week or weeks, for several months, or monthly or quarterly for many months. It is also possible to administer 2, 3, 4, 5 or 6 times continuously for each course of treatment, for example, at an interval of about 10 days, 15 days, 20 days, 25 days, 30 days, 35 days, 40 days, 45 days, 50, 55 days or 60 days or more after continuous administration for the next course of treatment.

In general, the dosage of other therapeutic agents for human administration will vary depending on factors such as the age, weight, height, sex, general medical condition, and past medical history of the patient. It may be desirable to provide the recipient with an antibody conjugate dose as a single intravenous infusion in the range of about 1 mg/kg to 25 mg/kg, but lower or higher doses may also be administered, as appropriate. For example, for a 70 kg patient, the dose of 1-20 mg/kg is 70-1,400 mg. The dose can be repeated as needed, for example, once a week for 4-10 weeks, once a week for 8 weeks or once a week for 4 weeks. In maintenance therapy, it can also be administered at a lower frequency, if desired, such as every other week for several months, or monthly or quarterly for many months.

Other therapeutic agents can be administered in one dose every 2 weeks or 3 weeks, for a total of at least 3 doses repeated. Alternatively, the combination can be administered twice a week for 4 to 6 weeks. If the dose is reduced to approximately 200-300 mg/m$^2$ (340 mg per dose for 1.7 m patients or 4.9 mg/kg for 70 kg patients), it may be administered once or even twice a week for 4 to 10 weeks. Alternatively, the time course of administration can be reduced, i.e., once every 2 or 3 weeks for 2-3 months. However, it has been determined that even higher doses can be administered by slow i.v. infusion, such as 20 mg/kg once a week or once every 2-3 weeks, for repeated dosing cycles. The time course of administration can optionally be repeated at other intervals, and the dosage can be administered by various parenteral routes with appropriate adjustments in dosage and time course.

In a preferred embodiment, the antibody conjugate or pharmaceutical composition described herein is useful in the treatment of cancer therapy. Examples of cancer include, but are not limited to, carcinoma, lymphoma, glioblastoma, melanoma, sarcoma and leukemia, myeloma or lymphoid malignancy. More specific examples of the cancer are described below and include: squamous cell carcinoma (e.g., epithelial squamous cell carcinoma), Ewing's sarcoma, Wilms' tumor, astrocytoma, lung cancer (including small cell lung cancer, non-small cell lung cancer, lung adenocarcinoma and lung squamous cell carcinoma), peritoneal cancer, hepatocellular cancer, gastric cancer or stomach cancer (including gastrointestinal cancer), pancreatic cancer, glioblastoma multiforme, cervical cancer, ovary cancer, liver cancer, bladder cancer, hepatocellular cancer, hepatocellular carcinoma, neuroendocrine tumor, medullary thyroid carcinoma, thyroid differentiation cancer, breast cancer, ovarian cancer, colon cancer, rectal cancer, endometrial cancer or uterine cancer, salivary gland cancer, renal or kidney cancer, prostate cancer, vulva cancer, anal cancer, penile cancer, and head and neck cancer. The term "cancer" includes primary malignant cell or tumor (e.g., a tumor where cells have not migrated to a site in a subject other than the original malignant disease or tumor site) and secondary malignant cells or tumors (e.g., a tumor produced by metastasis, i.e., malignant cells or tumor cells migrating to a secondary site different from the original tumor site). Cancers that benefit from the therapeutic method of the invention are directed to cells that express, overexpress, or aberrantly express IGF-IR.

Other examples of the cancer or malignant disease include, but are not limited to, acute childhood lymphoblastic leukemia, acute lymphoblastic leukemia, acute lymphocytic leukemia, acute myeloid leukemia, adrenocortical carcinoma, adult (primary) hepatocellular carcinoma, adult (primary) liver cancer, adult acute lymphocytic leukemia, adult acute myeloid leukemia, adult Hodgkin's lymphoma, adult lymphocytic lymphoma, adult non-Hodgkin's lymphoma, adult primary liver cancer, adult soft tissue sarcoma, AIDS-related lymphoma, AIDS-related malignant disease, anal cancer, astrocytoma, cholangiocarcinoma, bladder cancer, bone cancer, brain stem glioma, brain tumor, breast cancer, renal pelvis and ureteral cancer, central nervous system (primary) lymphoma, central nervous system lymphoma, cerebellar astrocytoma, cerebral astrocytoma, cervical cancer, children (primary) hepatocellular carcinoma, children (primary) liver cancer, children acute lymphoblastic leukemia, children acute myeloid leukemia, children brain stem glioma, children cerebellar astrocytoma, children cerebral astrocytoma, children extracranial blastoma, Hodgkin's disease in children, Hodgkin's lymphoma in children, hypothalamic and visual pathway glioma in children, lymphoblastic leukemia in children, medulloblastoma in children, non-Hodgkin's lymphoma in children, pineal gland and supratentorial primitive neuroectodermal tumor in children, children primary liver cancer, childhood rhabdomyosarcoma, childhood soft tissue sarcoma, childhood visual pathway and hypothalamic glioma, chronic lymphocytic leukemia, chronic myeloid leukemia, colon cancer, cutaneous T-cell lymphoma, endocrine islet cell carcinoma, endometrial cancer, ependymoma, epithelial cancer, esophageal cancer, Ewing's sarcoma and related tumors, exocrine pancreatic cancer, extracranial blastoma, extragonadal blastoma, extrahepatic cholangiocarcinoma, eye cancer, female breast cancer, Gaucher's disease, gallbladder cancer, stomach cancer, benign gastrointestinal tumor, gastrointestinal tumor, blastocytoma, gestational trophoblastic cell tumor, hairy cell leukemia, head and neck cancer, hepatocellular carcinoma, Hodgkin's lymphoma, hypergammaglobulinemia, hypopharyngeal carcinoma, intestinal cancer, intraocular melanoma, islet cell carcinoma, islet cell pancreatic cancer, Kaposi's sarcoma, kidney cancer, laryngeal cancer, lip and mouth cancer, liver cancer, lung cancer, lymphoproliferative disorders, macroglobulinemia, male breast cancer, malignant mesothelioma, malignant thymoma, medulloblastoma, melanoma, mesothelioma, metastatic primary occult squamous neck cancer, metastatic primary squamous neck cancer, metastatic squamous neck cancer, multiple myeloma, multiple myeloma/plasma neoplasms, myelodysplastic syndrome, myeloid leukemia, myelogenous leukemia, myeloproliferative disorders, nasal and paranasal sinus cancer, nasopharyngeal carcinoma, neuroblastoma, non-Hodgkin's lymphoma, non-melanoma skin cancer, non-small cell lung cancer, metastatic primary occult squamous neck cancer, oropharyngeal cancer, osteosarcoma/malignant fibrosarcoma, osteosarcoma/malignant fibrous histiocytoma, osteosarcoma/malignant fibrous histiocytoma in bone, ovarian epithelial carcinoma, ovarian blastoma, ovarian low malignant potential tumor, pancreatic cancer, diseased proteinemia, polycythemia vera, parathyroid carcinoma, penile cancer, pheochromocytoma, pituitary tumor, primary central nervous system lymphoma, primary liver cancer, prostate cancer, rectal cancer, renal cell carcinoma, renal pelvis and ureteral cancer, retinoblastoma, rhabdomyosarcoma, salivary gland cancer, sarcoidosis sarcoma, Sezary syndrome, skin cancer, small cell lung cancer, small bowel cancer, soft tissue sarcoma, squamous neck cancer, gastric cancer, supratentorial primitive neuroectodermal and pineal tumor, T cell lymphoma, testicular cancer, thymoma, thyroid cancer, renal pelvis and ureteral transitional cell carcinoma, transitional renal pelvis and ureteral cancer, trophoblastic tumor, ureter and renal pelvic cell carcinoma, ureteral cancer, uterine cancer, uterine sarcoma, vaginal cancer, visual pathway and hypothalamic glioma, vulva cancer, Waldenstrom's macroglobulinemia, Wilms' tumor, and any other hyperproliferative disease other than a neoplasma located in the organ system listed above.

The antibody conjugate, composition, and method described and claimed herein can be used to treat malignant or pre-malignant condition and to prevent progression to a neoplastic or malignant state, including but not limited to those described above. It is indicated that such use is related to conditions that are known or suspected to progress in advance into a neoplasma or cancer, especially in the case of non-neoplastic cell growth that has occurred from hyperplasia, metaplasia or, most particularly, dysplasia. For a review of such abnormal growth conditions, see Robbins and Angell, BASIC PATHOLOGY, 2nd ed., W.B. Saunders Co., Philadelphia, pp. 68-79 (1976).

Dysplasia is often a precursor to cancer and is primarily found in the epithelium. It is the most disordered form of non-neoplastic cell growth and involves the loss of individual cell consistency and cell structure orientation. In the presence of chronic irritation or inflammation, dysplasia characteristically occurs. Dysplastic disorders that can be treated include, but are not limited to, non-perspiring ectodermal dysplasia, anterior dysplasia, asphyxiating thoracic dysplasia, atrial-finger dysplasia, bronchopulmonary dysplasia, cerebral dysplasia, cervical dysplasia, cartilage ectodermal dysplasia, clavicular skull dysplasia, congenital ectodermal dysplasia, skull diaphysis dysplasia, cranium carpus tarus dysplasia, skull metaphyseal dysplasia, dentine dysplasia, diaphysis dysplasia, ectodermal dysplasia, dental enamel dysplasia, cerebral eyeball dysplasia, lateral epiphyseal dysplasia, multiple epiphyseal dysplasia, punctate epiphyseal dysplasia, epithelial dysplasia, facial phalangeal genital dysplasia, familial jaw fibrous dysplasia, familial white wrinkle dysplasia, fibromuscular dysplasia, bone fibrous dysplasia, vigorous bone dysplasia, hereditary renal retinal dysplasia, perspiring ectodermal dysplasia, hypohidrotic ectodermal dysplasia, lymphopenia thymic dysplasia, mammary gland dysplasia, mandibular face dysplasia, metaphyseal dysplasia, Montini's dysplasia, monofibrillar bone dysplasia, mucous epithelial dysplasia, multiple epiphyseal dysplasia, ocular aural vertebral dysplasia, ocular dental phalangeal dysplasia, ocular vertebral dysplasia, odontogenic dysplasia, ocular mandibular dysplasia, root tip periodontal cemental dysplasia, multifibrous bone dysplasia, pseudoachondroplasia vertebral epiphyseal dysplasia, retinal dysplasia, septal-ocular dysplasia, vertebral epiphyseal dysplasia and Cerebroventricular radial dysplasia.

Other pre-neoplastic disorders that can be treated include, but are not limited to, benign abnormal proliferative disorders (e.g., benign tumors, fibrocystic conditions, tissue hypertrophy, intestinal polyps or adenomas and esophageal dysplasia), leukoplakia, keratosis, Bowen's Disease, farmer's skin, solar cheilitis and solar keratosis.

In a preferred embodiment, the method of the invention is used to inhibit cancers, particularly the growth, progression and/or metastasis of the cancers listed above.

Other hyperproliferative diseases, disorders, and/or conditions include, but are not limited to, progression and/or metastasis of malignant diseases and related conditions, such as leukemia (including acute leukemia (e.g., acute lymphocytic leukemia, acute myeloid leukemia (including myeloblastic, promyelocytic, myelomonocytic, monocytic, and erythroleukemia)) and chronic leukemia (e.g., chronic myeloid (granulocytic) leukemia and chronic lymphocytic leukemia)), polycythemia vera, lymphoma (e.g., Hodgkin's disease and non-Hodgkin's disease), multiple myeloma, Waldenstrom's macroglobulinemia, heavy chain disease, and solid tumors, including but not limited to sarcoma and carcinoma, such as fibrosarcoma, mucinous sarcoma, liposarcoma, chondrosarcoma, osteosarcoma, chordoma, angiosarcoma, endothelial sarcoma, lymphangiosarcoma, lymphatic endothelial sarcoma, synovial tumor, mesothelioma, Ewing's Tumor, leiomyosarcoma, rhabdomyosarcoma, colon cancer, pancreatic cancer, breast cancer, ovarian cancer, prostate cancer, squamous cell carcinoma, basal cell carcinoma, adenocarcinoma, sweat gland cancer, sebaceous gland cancer, papillary carcinoma, papillary adenocarcinoma, cystadenocarcinoma, medullary carcinoma, bronchial carcinoma, renal cell carcinoma, hepatoma, cholangiocarcinoma, choriocarcinoma, seminoma, embryonal cancer, Wilms' tumor, cervical cancer, testicular cancer, lung cancer, small cell lung cancer, bladder cancer, epithelial cancer, glioma, astrocytoma, medulloblastoma, craniopharyngioma, ependymoma, pineal gland tumor, hemangioblastoma, acoustic neuroma, oligodendroglioma, meningioma, melanoma, neuroblastoma and retinoblastoma.

Kit

Various embodiments may involve a kit containing a component suitable for treating or diagnosing diseased tissue of a patient. An exemplary kit can contain one or more antibody conjugates, pharmaceutical compositions, leukocytes, and/or other therapeutic agents as described herein. If a composition containing multiple components for administration is not formulated for delivery via the digestive tract, such as by oral delivery, a device capable of delivering the components of the kit by some other routes may be included. One type of device for applications such as parenteral delivery is a syringe for injecting a composition into a subject. An inhalation device can also be used. In certain embodiments, the therapeutic agent can be provided in the form of a prefilled syringe or autoinjector pen containing a sterile liquid formulation or a lyophilized formulation.

The components of kit can be packaged together or separated into two or more containers. In some embodiments, the container can be a vial containing a sterile lyophilized formulation of a composition suitable for reconstitution. The kit may also contain one or more buffers suitable for reconstitution and/or dilution of other reagents. Other containers that may be used include, but are not limited to, bags, trays, boxes, tubes, and the like. The components of kit can be aseptically packaged and maintained in a container. Another component that can be included is the insert or leaflet for the kit.

**Examples**

The following examples are provided to illustrate the invention but not to limit the claims of the invention.

**Example 1. Preparation of anti-CD16 VHH-anti-MUC1 VHH bispecific** Nanobody

1. Design and Synthesis of the Nucleotide Sequence of Anti-CD16 VHH-Anti-MUC1 VHH Bispecific Nanobody

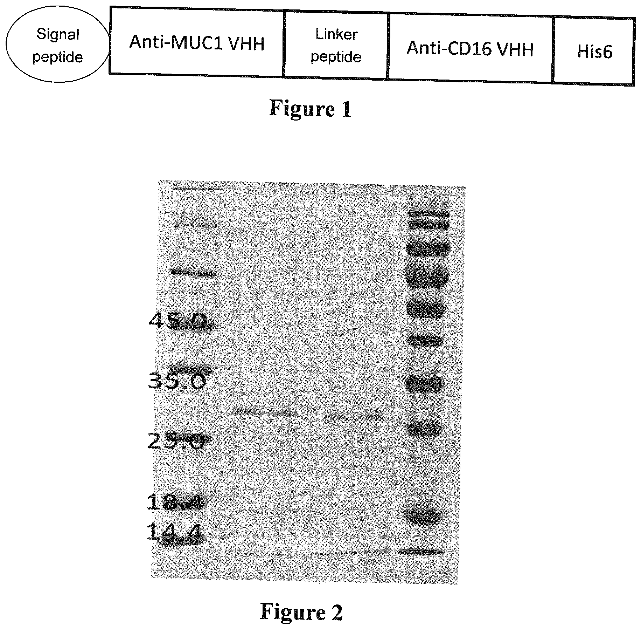

Anti-CD16 VHH-anti-MUC1 VHH bispecific Nanobody is hereinafter referred to as BiTE(CD16-MUC1), wherein the amino acid sequence of anti-MUC1 VHH is shown in SEQ ID NO: 1, the amino acid sequence of anti-CD16 VHH is shown in SEQ ID NO: 2, and the amino acid sequence of the linker peptide is shown in SEQ ID NO: 3. Based on the sequence and ligation format of the bispecific antibody, the nucleotide sequence was redesigned and optimized, and a NcoI restriction site was added at the 5' end of the sequence, and a Hind III restriction site was added at the 3' end. DsbA-anti-MUC1 VHH-GS-anti-CD16 VHH-6His was directly synthesized and ligated into the vector pETDuet by double digestion to form an expression plasmid. A schematic diagram of the structure of BiTE(CD16-MUC1) is shown in FIG. 1.

2. Vector transformation of BL21 (DE3)

The plasmid was transformed into *E. coli* DH5α competent cell line. Positive clones were selected, cultured in LB medium (3 mL) containing 100 μg/ml ampicillin overnight at 37 °C. The bacteria solution was centrifuged at 5000 rpm at RT and the supernatant was discarded. The obtained *E. coli* was lysed and extracted for the plasmid with Qiagen's plasmid extraction kit to obtain the expression vector.

3. Expression and purification of BiTE(CD16-MUC1)

The purified pETDuet-bsAb expression vector was transformed into *E. coli* BL21 (DE3) strain. Positive clones were selected and cultured in LB medium (3 mL) containing 100 μg/mL ampicillin overnight at 37 °C, and transferred to 300 mL LB containing 100 μg/mL ampicillin, and cultured at 37 °C to an OD600 of 0.6-0.8. IPTG was added to a final concentration of 0.05 mM, and the culture was induced at 16 °C for 16 hours. The supernatant was discarded by centrifuging at 4000 rpm, and the pellet was added with a solution of 20 mM Tris-HCl, pH 8.0, 25% sucrose, 1 mM EDTA in a weight/volume ratio of 1:4, resuspended and then ice bathed for 30 min, and centrifuged at 8500 g at 4 °C for 20 minutes. The supernatant was left. The pellet was added with 5 mM MgCl$_2$, 1 mg/mL lysozyme solution in a weight/volume ratio of 1:5, resuspended, ice bathed for 20 min, and centrifuged at 8500 g, at 4 °C for 20 minutes, and the supernatant was removed.

Binding: Two supernatants were pooled and passed through a 2 mL Ni-NTA (Qiagen) gravity settling column.

Removal of impurities: The impurities were removed by 20 mL of 20 mM Tris-HCl pH 8.0, 15 mM imidazole, 1 M NaCl and 20 mL of 20 mM Tris-HCl pH 8.0, 25 mM imidazole, 1 M NaCl, sequentially.

Elution: Elution was carried out with 10 mL of 20 mM Tris-HCl pH 8.0, 50 mM imidazole, 0.15 M NaCl, 10 mL of 20 mM Tris-HCl pH 8.0, 100 mM imidazole, 0.15 M NaCl, 10 mL of 20 mM Tris-HCl pH 8.0, 200 mM imidazole, 0.15 M NaCl, and 10 of mL 20 mM Tris-HCl pH 8.0, 500 mM imidazole, 0.15 M NaCl, sequentially.

The fractions eluted with 50 mM imidazole, 100 mM imidazole, and 200 mM imidazole were pooled, and dialyzed against 20 mM PB, pH 7.6, 10% glycerol at 4 °C overnight.
1 mL Q-HP purification: After dialysis overnight, the solution comprising the target component was centrifuged at 20,000 g at 4 °C for 20 min, and then loaded at 1 mL/min flow rate, and the flow-through components were collected for ultrafiltration concentration.
The experimental results are shown in Figure 2.
After *in vitro* test (flow cytometry and Western blot) for binding to MUC1, the bispecific antibody BiTE(CD16-MUC1) was shown to specifically bind to MUC1 expressed by cells such as LS174T, HT29 and SKOV3, but not to total protein in CHO and HepG2 (data not shown).

**Example 2. Preparation of anti-CD16 VHH-anti-CEA VHH bispecific Nanobody**

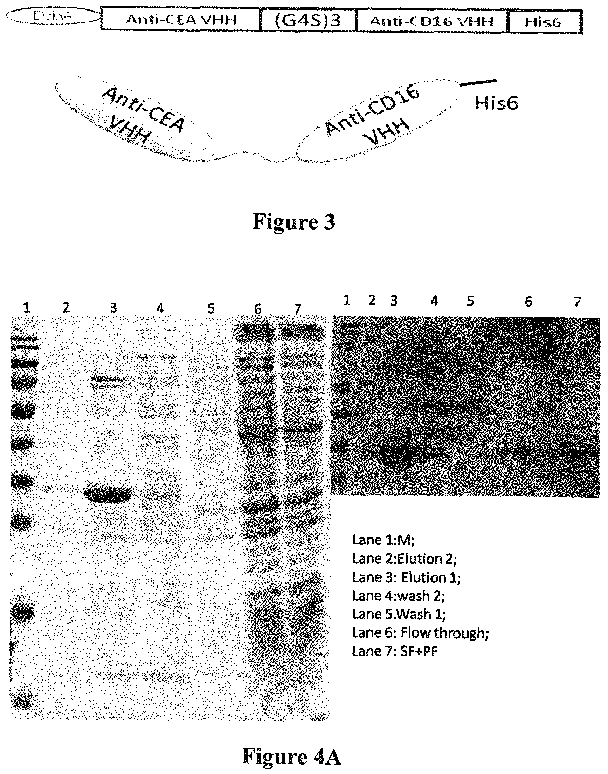

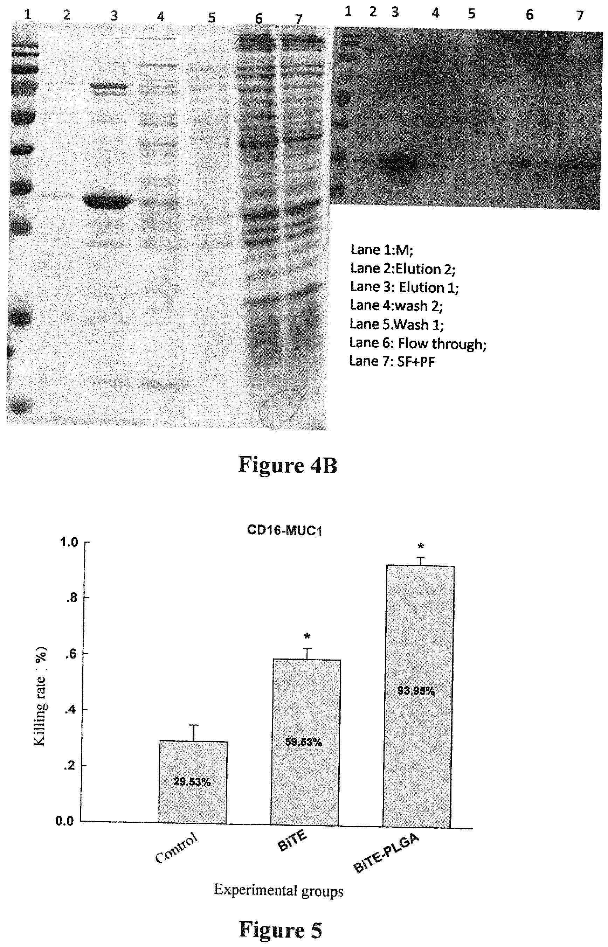

The anti-CD 16 VHH-anti-CEA VHH bispecific Nanobody is hereinafter referred to as BiTE(CD16-CEA), and a schematic diagram of the structure thereof is shown in Figure 3, wherein the amino acid sequence of anti-CEA VHH is shown in SEQ ID NO: 4, and the amino acid sequence of anti-CD16 VHH is shown in SEQ ID NO: 5. Based on the sequence and ligation format of the bispecific antibody, the nucleotide sequence was redesigned and optimized, and a NcoI restriction site was added at the 5' end of the sequence, and a Hind III restriction site was added at the 3' end. DsbA-anti-CEA VHH-(GGGGS)3-anti-CD16 VHH-6His was directly synthesized and ligated into the vector pETDuet by double digestion to form a pETDuet-bsAb expression plasmid.
Vector transformation and expression and purification of BiTE(CD16-CEA) were carried out according to the method described in Example 1. The experimental results are shown in Figure 4.
After *in vitro* test (flow cytometry and Western blot) for binding to MUC1, the bispecific antibody BiTE(CD16-MUC1) was shown to specifically bind to CEA expressed by cells such as LS174T, HT29 and SKOV3-CEA, but not to total protein in SKOV3. The positive control had the same experimental results (data not shown).

**Example 3. Preparation of bispecific antibody conjugates**

Bispecific antibody conjugates (BiTE(CD16-MUC1)-PLGA and BiTE(CD16-CEA)-PLGA) having BiTE(CD16-MUC1) and BiTE(CD16-CEA) conjugated to PLGA nanoparticles, respectively, were prepared.
The specific method for preparing the bispecific antibody conjugates is as follows:

(1) preparing PLGA nanoparticles: completely dissolving the PLGA in acetone at a concentration of 5 mg/mL, and adding the solution of PLGA in acetone into deionized water in a volume ratio of 1:4 of acetone and deionized water with magnetic stirring at 1000 rpm/min, to form a uniform emulsion, and then continually stirring until volatilization of acetone;

(2) collecting the PLGA nanoparticles: collecting the prepared nanoparticles with larger particle sizes by centrifugation at 8000 rpm/min for 10min; then collecting the prepared nanoparticles with smaller particle sizes by centrifugation at 15000 rpm/min for 10 min, discarding the nanoparticles with larger particle sizes and resuspending the nanoparticles with smaller particle sizes in deionized water, and repeating the process twice to wash the nanoparticles. The nanomaterials may be further purified to obtain smaller nanoparticles. The nanoparticles with smaller particle sizes are used in the process as follows;

(3) activating the PLGA nanoparticles: using a mixed solvent of 5 mg/mL 1-ethyl-(3-dimethylaminopropyl)carbodiimide hydrochloride and N-hydroxysuccinimide at room temperature to activate the PLGA nanoparticles for 1h;

(4) ligating the PLGA nanoparticles to antibodies: collecting the activated nanomaterials by centrifugation, and then washing the nanomaterials once with 0.1 M D-PBS at pH = 8.0, adding BiTE(CD16-MUC1) or BiTE(CD16-CEA) to be ligated to the ligation reaction solution, then resuspending the nanomaterials with the ligation reaction solution containing the BiTE, and conducting the ligating reaction for 0.5h at room temperature. After the reaction, the nanomaterials are collected by centrifugation. The nanomaterials are washed twice in D-PBS, and then resuspended in D-PBS and stored at 4 °C before use.

**Example 4. Efficacy of bispecific antibody conjugates on killing cancer cells**

The ability of the bispecific antibody conjugates prepared in Example 3 to kill tumor cells was evaluated.
Specifically, 5000 target cells per well were cultured for 12 h in 96-well plates and then the initial medium was discarded. The cytokine-free X-vivo 15 medium (purchased from lonza) was used to adjust the density of NK cells (obtained from amplification induced by peripheral blood mononuclear cells from healthy blood donors), so that number of NK cells in a volume of 100 μl reached 4 times larger than that of the target cells (effector to target ratio is 4:1). 100 μl suspension of NK cells was added in a cancer cell culture plate, and 10 μl prepared bispecific antibody conjugate (bispecific antibody conjugate content: 0.2 mg, containing BiTE 0.2 μg) or bispecific antibody (BiTE(CD16-MUC1) or BiTE(CD16-CEA)) (0.2 μg) was added, incubated for 8 h in an incubator, and then CCK-8 reagent was added, incubated according to the reagent instruction. The absorbance at 450 nm was measured using a microplate reader. Statistical analysis of the data was performed, and the killing rate of DC-CIK cells on cancer cells was calculated according to the following formula.

$$\text{Killing rate} = [1- (\text{experimental group} - \text{effector control group}) / (\text{target control group} - \text{blank control group})] \times 100\%$$

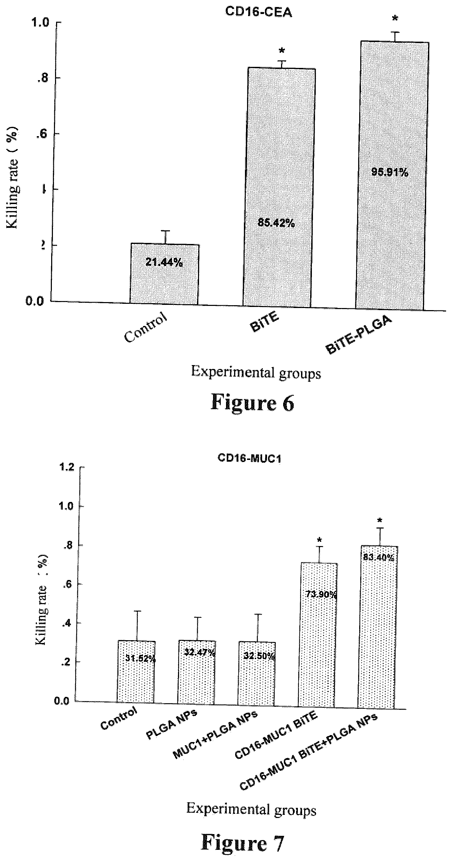

wherein the blank control group represents the added medium; the target control group represents the added target cells + medium; the effector control group represents the added effector cells + medium; the experimental group represents the added effector cells + target cells + medium + bispecific antibody conjugate.
The results are shown in Table 1 and Figures 5-6. Figure 5 shows the killing rate of the bispecific antibody BiTE(CD16-MUC1) and the antibody conjugate BiTE(CD16-MUC1)-PLGA in combination with NK cells on lung cancer cell A549 (human non-small cell lung cancer cells). Figure 6 shows the killing rate of the bispecific antibody BiTE(CD16-CEA) and the antibody conjugate BiTE(CD16-CEA)-PLGA in combination with NK cells on intestinal cancer cell HCT116. In those figures, the control represents the added effector cells + target cells + medium; BiTE represents the added effector cells + target cells + medium + BiTE; BiTE-PLGA represents the added effector cells + target cells + medium + BiTE-PLGA (i.e., bispecific antibody conjugate), wherein the PLGA nanoparticles have an average particle size of about 50 nm.

Table 1. Comparison of the ability of the bispecific antibodies and bispecific antibody conjugates on killing tumor cells (effector-target ratio of 4:1)

| Cancer cell | Killing rate | | |
|---|---|---|---|
| | Effector control group (NK) | Experimental group | |
| | | BiTE(CD16-MUC1) | BiTE(CD16-MUC1)-PLGA |
| A549 | 29.53% | 59.53% | 93.95% |
| | | | |

(continued)

|  | | BiTE(CD16-CEA) | BiTE(CD16-CEA)-PLGA |
|---|---|---|---|
| HCT116 | 21.44% | 85.42% | 95.91% |

**Example 5. Efficacy of bispecific antibody conjugates on killing cancer cells**

Bispecific antibody conjugates having a PLGA nanoparticle average particle size of about 100-150 nm were prepared according to the method of Example 3 using the same bispecific antibodies. In addition, the conjugate of Muc1 VHH (the sequence of which is shown in SEQ ID NO: 1) was prepared as a control using the same PLGA nanoparticles.
The ability of the bispecific antibody conjugates as prepared to kill tumor cells was evaluated.
Specifically, 5000 target cells per well were cultured for 12 h in 96-well plates and then the initial medium was discarded. The cytokine-free X-vivo 15 medium (purchased from lonza) was used to adjust the density of NK cells (obtained from amplification induced by peripheral blood mononuclear cells from healthy blood donors), so that number of NK cells in a volume of 100 μl reached 4 times larger than that of the target cells (effector to target ratio is 4:1). 100 μl suspension of NK cells was added in a cancer cell culture plate, and 10 μl prepared bispecific antibody conjugate CD16-MUC1 BiTE+PLGA NPs (conjugate content: 0.2 mg, containing CD16-MUC1 BiTE 0.2 μg) or bispecific antibody (BiTE) (0.2 μg) was added, or Muc1+PLGA NPs (conjugate content: 0.2 mg, containing Muc1 VHH 0.1 μg) as a control or PLGA nanoparticles, i.e., PLGA NPs (0.2 mg), were added. They were incubated for 48 h in an incubator, and then CCK-8 reagent was added, incubated according to the reagent instruction. The absorbance at 450 nm was measured using a microplate reader. Statistical analysis of the data was performed, and the killing rate of DC-CIK cells on cancer cells was calculated according to the following formula.

$$\text{Killing rate} = [1- (\text{experimental group} - \text{effector control group}) / (\text{target control group} - \text{blank control group})] \times 100\%$$

wherein the blank control group represents the added medium; the target control group represents the added target cells + medium; the effector control group represents the added effector cells + medium; the experimental group represents the added effector cells + target cells + medium + bispecific antibody conjugate.
The results are shown in Table 2 and Figure 7. Table 2 and Figure 7 show the killing rate of the bispecific antibody BiTE and the antibody conjugate BiTE+PLGA in combination with NK cells on lung cancer cell A549 (human non-small cell lung cancer cells), wherein the control represents the added effector cells + target cells + medium; MUC1 + PLGA NPs represents the added effector cells + target cells + medium + PLGA nanoparticles; BiTE represents the added effector cells + target cells + medium + bispecific antibody; BiTE-PLGA NPs represents the added effector cells + target cells + medium + bispecific antibody conjugate.

Table 2. The ability of the bispecific antibodies or bispecific antibody conjugates on killing tumor cell A549 (effector-target ratio of 4:1)

| Experimental groups | Killing rate |
|---|---|
| control | 31.52% |
| PLGA nanoparticles (PLGA NPs) | 32.47% |
| MUC1 + PLGA NPs | 32.50% |
| CD16-MUC1 BiTE | 73.90% |
| CD16-MUC1 BiTE + PLGA NPs | 83.40% |

**Example 6. Stability of bispecific antibody conjugates**

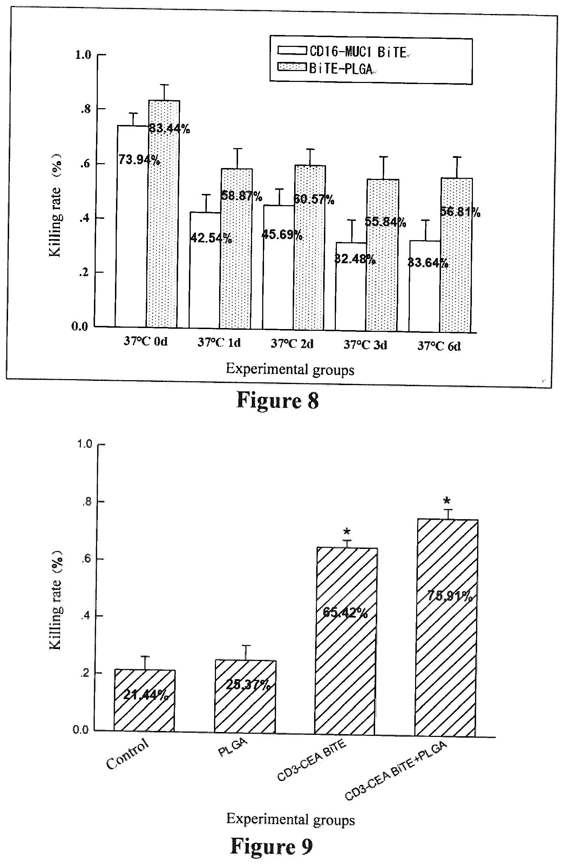

In the experiment described in Example 5, the stability of the bispecific antibody and its conjugate was tested simultaneously. Specifically, the bispecific antibody (CD16-MUC1 BiTE) and the bispecific antibody conjugate (BiTE-PLGA) having a PLGA nanoparticle average particle size of about 100-150 nm were placed at 37 °C for several days. The killing rate of the antibody and antibody conjugate having been placed for different times at 37 °C in combination with NK cells on lung cancer cell A549 (human non-small cell lung cancer cells) were tested. The results of the stability experiment are shown in Figure 8 (results represents three or more experiments, using spss 16.0, T test, *p < 0.05, ** p < 0.01).

The results shown in Figure 8 indicate that the activity of the bispecific antibody modified by the nanoparticles (antibody conjugate) still remained after 6 days (6d) under the condition of simulated human body temperature of 37 °C, and the killing rate thereof in combination with NK cells on A549 reached 56.81%. In contrast, the killing rate of the same amount of unmodified bispecific antibody group was only 33.64%, which was not significantly different from the killing rate of 31.52% when no bispecific antibody was added, indicating that the activity thereof had been lost. Therefore, the bispecific antibody modified by the nanoparticles had significantly improved stability.

**Example 7. Efficacy of the bispecific antibody conjugate in combination with T cells on killing cancer cells**

A bispecific antibody CD3-CEA BiTE (ie CEA-S-Fab) was prepared wherein the binding moiety that specifically binds to CD3 is a Fab fragment of an anti-CD3 antibody, and the binding moiety that specifically binds to CEA is the single domain antibody moiety.

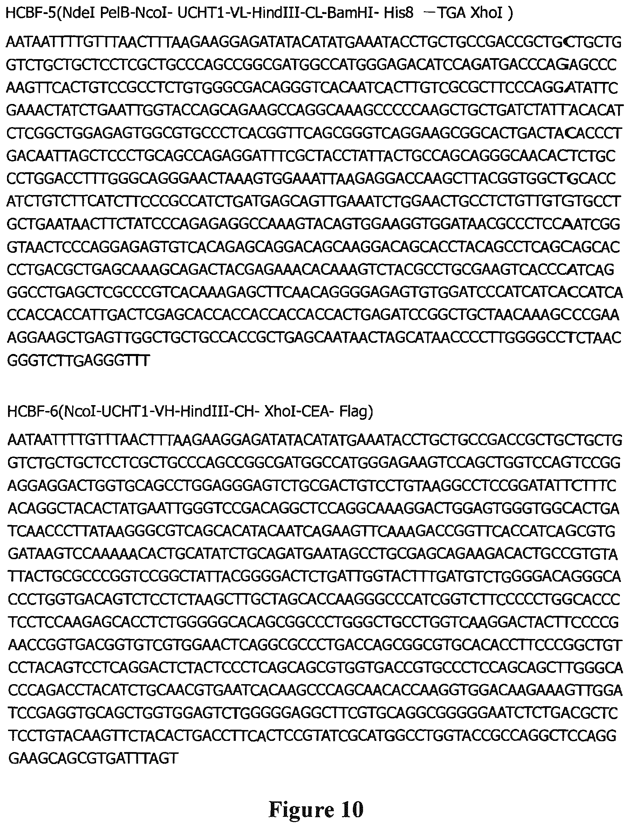

Briefly, in order to construct CEA-S-Fab, an anti-CEA single domain antibody was linked to the C-terminus of an anti-CD3 VH-CH1 through genetic engineering (Behar G et al., Protein Eng Des Sel. 2008; 21(1): 1 - 10). The target fragment anti-CD3 VH-CH1-CEA VHH (i.e., HCBF-6(NcoI-UCHT1-VH-HindIII-CH- XhoI-CEA- Flag), see Figure 10) and anti-CD3 VL-CL (i.e., HCBF-6(NcoI-UCHT1-VH-HindIII-CH-XhoI-CEA- Flag), see Figure 10) were cloned into vectors and transformed into BL21 (DE3) for co-expression, respectively. CEA-S-Fab was formed by heterodimerization of an anti-CD3 VH-CH1-CEA VHH with an anti-CD3 VL-CL polypeptide. CEA-S-Fab was extracted from the cell periplasm and purified by Ni-NTA Sepharose affinity purification.

To determine if CEA-S-Fab was correctly formed as a heterodimer, gel filtration was performed to analyze the purified CEA-S-Fab. The molecular weight of the purified CEA-S-Fab was determined to correspond to the expected molecular weight of CEA-S-Fab (about 65 kD), indicating that most of the CEA-S-Fab was correctly folded into a heterodimer.

Bispecific antibody conjugate CD3-CEA BiTE+PLGA NPs having a PLGA nanoparticle average particle size of about 100-150 nm were prepared according to the method of Example 3 using the prepared bispecific antibody. In addition, the ability of the prepared bispecific antibody and bispecific antibody conjugate to kill tumor cells was evaluated using the same PLGA nanoparticles as a control.

Specifically, 5000 target cells per well were cultured for 12 h in 96-well plates and then the initial medium was discarded. The cytokine-free X-vivo 15 medium was used to adjust the density of DC-CIK cells (obtained from amplification induced by peripheral blood mononuclear cells from healthy blood donors), so that number of DC-CIK cells in a volume of 100 $\mu$l reached 4 times larger than that of the target cells (effector to target ratio is 4:1). 100 $\mu$l suspension of DC-CIK cells was added in a cancer cell culture plate, and 10 $\mu$l prepared bispecific antibody (the bispecific antibody usage amount: 0.28 $\mu$g) or antibody conjugate (containing 0.28 $\mu$g of the bispecific antibody) was added, and incubated for 8 h in an incubator, and then CCK-8 reagent was added, incubated according to the reagent instruction. The absorbance at 450 nm was measured using a microplate reader. Statistical analysis of the data was performed, and the killing rate of DC-CIK cells on lung cancer cell A549 (human non-small cell lung cancer cells) was calculated according to the following formula.

$$\text{Killing rate} = [1- (\text{experimental group} - \text{effector control group}) / (\text{target control group} - \text{blank control group})] \times 100\%$$

wherein the blank control group represents the added medium; the target control group represents the added target cells + medium; the effector control group represents the added effector cells + medium; the experimental group represents the effector cells + target cells + medium + bispecific antibody.

Figure 9 shows the killing rate of the bispecific antibody BiTE and the antibody conjugate BiTE-PLGA in combination with T cells;

wherein control represents the added effector cells + target cells + medium; "PLGA" represents the added effector cells + target cells + medium + PLGA nanoparticles; "CD3-CEA BiTE" represents the added effector cells + target cells + medium + bispecific antibody; "CD3-CEA BiTE+PLGA NPs" represents the added effector cells + target cells + medium + bispecific antibody conjugate.

The results shown in Figure 9 indicate that the killing rate of the bispecific antibody conjugate in combination with T cells on cancer cells (75.91%) is higher than that of the bispecific antibody itself in combination with T cells on cancer cells (65.42%).

The experimental results provided by the present disclosure indicate that the conjugation of the bispecific antibody BiTE to the nanomaterial significantly increases the killing rate of BiTE in combination with NK cells on tumor cells or cancer cells. This effect is an unexpected technical effect for those of ordinary skill in the art.

While not wishing to be bound by theory, the inventors hypothesize that, as compared with unconjugated bispecific antibodies, due to multiple bispecific antibodies conjugated to conjugates, they bind to cytotoxic effector cells and target cells more strongly and may have a stronger stimulation to cytotoxic effector cells, resulting in an increase in the killing rate on target cells.

In addition, the experimental results also indicate that the conjugation of bispecific antibodies to nanomaterials also significantly improves their stability. It is expected that antibody conjugates will have a longer half-life *in vivo* as compared to bispecific antibodies such as BiTE, and thus it is expected that bispecific antibody conjugates will be more effective in animals or in humans than bispecific antibodies.

In accordance with the present disclosure, all of the conjugates, compositions, and methods disclosed and claimed herein can be made and used without undue experimentation. Although the compositions and methods have been described in terms of the preferred embodiments, it will be apparent to those skilled in the art that the conjugates, compositions and methods described herein as well as the steps or step sequences in the methods can vary without departing from the concept, spirit and scope of the invention. More specifically, certain reagents that are chemically and physiologically related may be substituted for the reagents described herein while achieving the same or similar results. All such similar substitutions and modifications which are obvious to those skilled in the art, are within the spirit, scope and concept of the invention as defined by the appended claims.

## Claims

1. An antibody conjugate comprising a multispecific antibody conjugated to a nanomaterial, preferably a biodegradable nanomaterial, such as a nanoparticle.

2. The antibody conjugate according to claim 1, wherein the multispecific antibody comprises a binding moiety that specifically binds to a cytotoxic effector cell.

3. The antibody conjugate according to claim 2, wherein the cytotoxic effector cell comprises a T cell and/or a NK cell.

4. The antibody conjugate according to any of claims 1-3, wherein the antibody comprises a binding moiety that specifically binds to a target cell, preferably a disease-associated cell.

5. The antibody conjugate according to claim 4, wherein the disease is a tumor or a cancer.

6. The antibody conjugate according to claim 4, wherein the disease is an inflammatory disease and/or an autoimmune disease.

7. The antibody conjugate according to any of claims 1-6, wherein the antibody is a bispecific antibody.

8. The antibody conjugate according to any of the preceding claims, wherein the nanomaterial is any one of or a mixture of at least two of poly(lactic acid-co-glycolic acid), polylactic acid, polycaprolactone, polybutylene succinate, polyaniline, polycarbonate, poly(glycolide-co-lactide) and poly(glycolide-co-caprolactone).

9. A pharmaceutical composition comprising the antibody conjugate of any of claims 1-8.

10. Use of the antibody conjugate of any of claims 1 to 8, or the pharmaceutical composition of claim 9 for the preparation of an immunotherapeutic medicament.

| Signal peptide | Anti-MUC1 VHH | Linker peptide | Anti-CD16 VHH | His6 |

**Figure 1**

**Figure 2**

Figure 3

Lane 1:M;
Lane 2:Elution 2;
Lane 3: Elution 1;
Lane 4:wash 2;
Lane 5.Wash 1;
Lane 6: Flow through;
Lane 7: SF+PF

Figure 4A

Lane 1:M;
Lane 2:Elution 2;
Lane 3: Elution 1;
Lane 4:wash 2;
Lane 5.Wash 1;
Lane 6: Flow through;
Lane 7: SF+PF

**Figure 4B**

CD16-MUC1

**Figure 5**

Figure 6

Figure 7

**Figure 8**

**Figure 9**

HCBF-5(NdeI PelB-NcoI- UCHT1-VL-HindIII-CL-BamHI- His8 —TGA XhoI )

AATAATTTTGTTTAACTTTAAGAAGGAGATATACATATGAAATACCTGCTGCCGACCGCTGCTGCTG
GTCTGCTGCTCCTCGCTGCCCAGCCGGCGATGGCCATGGGAGACATCCAGATGACCCAGAGCCC
AAGTTCACTGTCCGCCTCTGTGGGCGACAGGGTCACAATCACTTGTCGCGCTTCCAGGATATTC
GAAACTATCTGAATTGGTACCAGCAGAAGCCAGGCAAAGCCCCCAAGCTGCTGATCTATTACACAT
CTCGGCTGGAGAGTGGCGTGCCCTCACGGTTCAGCGGGTCAGGAAGCGGCACTGACTACACCCT
GACAATTAGCTCCCTGCAGCCAGAGGATTTCGCTACCTATTACTGCCAGCAGGGCAACACTCTGC
CCTGGACCTTTGGGCAGGGAACTAAAGTGGAAATTAAGAGGACCAAGCTTACGGTGGCTGCACC
ATCTGTCTTCATCTTCCCGCCATCTGATGAGCAGTTGAAATCTGGAACTGCCTCTGTTGTGTGCCT
GCTGAATAACTTCTATCCCAGAGAGGCCAAAGTACAGTGGAAGGTGGATAACGCCCTCCAATCGG
GTAACTCCCAGGAGAGTGTCACAGAGCAGGACAGCAAGGACAGCACCTACAGCCTCAGCAGCAC
CCTGACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGTGGATCCCATCATCACCATCA
CCACCACCATTGACTCGAGCACCACCACCACCACCACTGAGATCCGGCTGCTAACAAAGCCCGAA
AGGAAGCTGAGTTGGCTGCTGCCACCGCTGAGCAATAACTAGCATAACCCCTTGGGGCCTCTAAC
GGGTCTTGAGGGTTT

HCBF-6(NcoI-UCHT1-VH-HindIII-CH- XhoI-CEA- Flag)

AATAATTTTGTTTAACTTTAAGAAGGAGATATACATATGAAATACCTGCTGCCGACCGCTGCTGCTG
GTCTGCTGCTCCTCGCTGCCCAGCCGGCGATGGCCATGGGAGAAGTCCAGCTGGTCCAGTCCGG
AGGAGGACTGGTGCAGCCTGGAGGGAGTCTGCGACTGTCCTGTAAGGCCTCCGGATATTCTTTC
ACAGGCTACACTATGAATTGGGTCCGACAGGCTCCAGGCAAAGGACTGGAGTGGGTGGCACTGA
TCAACCCTTATAAGGGCGTCAGCACATACAATCAGAAGTTCAAAGACCGGTTCACCATCAGCGTG
GATAAGTCCAAAAACACTGCATATCTGCAGATGAATAGCCTGCGAGCAGAAGACACTGCCGTGTA
TTACTGCGCCCGGTCCGGCTATTACGGGGACTCTGATTGGTACTTTGATGTCTGGGGACAGGGCA
CCCTGGTGACAGTCTCCTCTAAGCTTGCTAGCACCAAGGGCCCATCGGTCTTCCCCCTGGCACCC
TCCTCCAAGAGCACCTCTGGGGGCACAGCGGCCCTGGGCTGCCTGGTCAAGGACTACTTCCCCG
AACCGGTGACGGTGTCGTGGAACTCAGGCGCCCTGACCAGCGGCGTGCACACCTTCCCGGCTGT
CCTACAGTCCTCAGGACTCTACTCCCTCAGCAGCGTGGTGACCGTGCCCTCCAGCAGCTTGGGCA
CCCAGACCTACATCTGCAACGTGAATCACAAGCCCAGCAACACCAAGGTGGACAAGAAAGTTGGA
TCCGAGGTGCAGCTGGTGGAGTCTGGGGGAGGCTTCGTGCAGGCGGGGGAATCTCTGACGCTC
TCCTGTACAAGTTCTACACTGACCTTCACTCCGTATCGCATGGCCTGGTACCGCCAGGCTCCAGG
GAAGCAGCGTGATTTAGT

## Figure 10

## INTERNATIONAL SEARCH REPORT

| International application No. |
| --- |
| PCT/CN2018/073785 |

**A. CLASSIFICATION OF SUBJECT MATTER**

C07K 17/08 (2006.01) i; C07K 16/46 (2006.01) i; A61K 39/44 (2006.01) i; A61K 47/59 (2017.01) i; A61K 47/60 (2017.01) i; A61P 35/00 (2006.01) i; A61P 37/02 (2006.01) i; A61P 29/00 (2006.01) i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CPRSABS; CNKI; CNTXT; DWPI; SIPOABS; EPTXT; USTXT; WOTXT; JPTXT; ELSEVIER; CA and search terms: 抗体, 偶联物, 纳米材料, 生物降解, 聚乳酸-羟基乙酸, 聚乳酸, 聚己内酯, 多特异性抗体, 双特异性抗体, T 细胞, NK 细胞, 肿瘤, 抗原, antibody, conjugate, nanomaterials, nanoparticle, biodegradable, BsAb, PLGA, PLA, PCL, polyspecific antibodies, bispecific antibodies, T cell, NK cell, tumor, antigen 等;

NATIONAL BIO-SEQUENCE DATABASE OF CHINESE PATENT; Genbank; EMBL; STN and searched sequence: SEQ ID NO: 1-5 等

**C. DOCUMENTS CONSIDERED TO BE RELEVANT**

| Category* | Citation of document, with indication, where appropriate, of the relevant passages | Relevant to claim No. |
| --- | --- | --- |
| PX | WO 2017219974 A1 (BENHEALTH BIOPHARMACEUTICAL (SHENZHEN) CO., LTD.), 28 December 2017 (28.12.2017), see claims 1-15 | 1-10 |
| X | WO 2016165632 A1 (SHENZHEN ZHONGLIAN BIOTECHNOLOGY CO., LTD.), 20 October 2016 (20.10.2016), see the abstract, and claims 1-17 | 1-10 |

☐ Further documents are listed in the continuation of Box C.   ☒ See patent family annex.

| | |
| --- | --- |
| * Special categories of cited documents:<br><br>"A" document defining the general state of the art which is not considered to be of particular relevance<br><br>"E" earlier application or patent but published on or after the international filing date<br><br>"L" document which may throw doubts on priority claim(s) or which is cited to establish the publication date of another citation or other special reason (as specified)<br><br>"O" document referring to an oral disclosure, use, exhibition or other means<br><br>"P" document published prior to the international filing date but later than the priority date claimed | "T" later document published after the international filing date or priority date and not in conflict with the application but cited to understand the principle or theory underlying the invention<br><br>"X" document of particular relevance; the claimed invention cannot be considered novel or cannot be considered to involve an inventive step when the document is taken alone<br><br>"Y" document of particular relevance; the claimed invention cannot be considered to involve an inventive step when the document is combined with one or more other such documents, such combination being obvious to a person skilled in the art<br><br>"&" document member of the same patent family |
| Date of the actual completion of the international search<br><br>21 March 2018 | Date of mailing of the international search report<br><br>12 April 2018 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br><br>HE, Rui<br><br>Telephone No. (86-10) 62411106 |

Form PCT/ISA/210 (second sheet) (July 2009)

## INTERNATIONAL SEARCH REPORT
### Information on patent family members

International application No.

PCT/CN2018/073785

| Patent Documents referred in the Report | Publication Date | Patent Family | Publication Date |
|---|---|---|---|
| WO 2017219974 A1 | 28 December 2017 | None | |
| WO 2016165632 A1 | 20 October 2016 | WO 2016165302 A1 | 20 October 2016 |
| | | CN 107531790 A | 02 January 2018 |
| | | US 2018021440 A1 | 25 January 2018 |
| | | CN 104829730 A | 12 August 2015 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014180577 A
- CN 106188305 A
- CN 101534865 A

**Non-patent literature cited in the description**

- **ANSEL et al.** PHARMACEUTICAL DOSAGE FORMS AND DRUG DELIVERY SYSTEMS. Lea & Febiger, 1990
- REMINGTON'S PHARMACEUTICAL SCIENCES. Mack Publishing Company, 1990
- **SHERWOOD et al.** *Bio/Technology,* 1992, vol. 10, 1446
- **SALTZMAN et al.** *Biophys. J.,* 1989, vol. 55, 163
- **ROBBINS ; ANGELL.** BASIC PATHOLOGY. W.B. Saunders Co, 1976, 68-79
- **BEHAR G et al.** *Protein Eng Des Sel.,* 2008, vol. 21 (1), 1-10